(19) Europäisches Patentamt
European Patent Office
Office européen des brevets

(11) Numéro de publication : **0 454 587 A1**

(12) **DEMANDE DE BREVET EUROPEEN**

(21) Numéro de dépôt : **91401111.9**

(22) Date de dépôt : **26.04.91**

(51) Int. Cl.$^5$ : **C07D 311/22**, A61K 31/35, C07D 493/04, // (C07D493/04, 311:00, 307:00)

(30) Priorité : **27.04.90 FR 9005360**

(43) Date de publication de la demande :
**30.10.91 Bulletin 91/44**

(84) Etats contractants désignés :
**AT BE CH DE DK ES FR GB GR IT LI LU NL SE**

(71) Demandeur : **ADIR ET COMPAGNIE**
**1 rue Carle Hébert**
**F-92415 Courbevoie Cédex (FR)**

(72) Inventeur : **Brion, Jean Daniel**
**76 rue du Château**
**F-78160 Saint-Leu la Foret (FR)**
Inventeur : **Le Baut, Guillaume**
**5 rue de la Baugerie**
**F-44230 Saint-Sebastien sur Loire (FR)**
Inventeur : **Zammattio, Françoise**
**11 boulevard des Martyrs Nantais**
**F-44100 Nantes (FR)**
Inventeur : **Pierre, Alain**
**52 rue de Monval**
**F-78160 Marly le Roi (FR)**
Inventeur : **Atassi, Ghanem**
**4 rue Joséphine**
**F-92210 Saint Cloud (FR)**
Inventeur : **Belachmi, Larbi**
**38 rue Antoine Watteau**
**F-44100 Nantes (FR)**

(54) **Nouveaux dérivés hétérocycliques : 2-styryl 4H-1-benzopyrane-4-ones, leur procédé de préparation et les compositions pharmaceutiques qui les contiennent.**

(57) Composés de formule générale (I) :

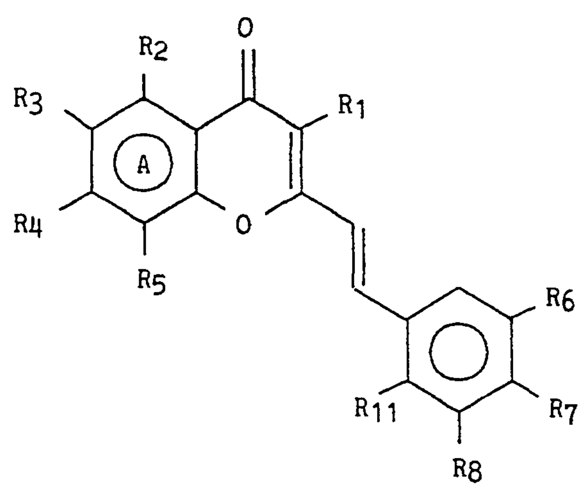

(I)

dans laquelle :

$R_1$ représente un atome d'hydrogène, un groupement alcoyle inférieur, hydroxyle ou alcoyloxyle inférieur,

$R_2$, $R_3$, $R_4$, $R_5$, $R_6$, $R_7$, $R_8$ et $R_{11}$ définis dans la description.

Médicaments.

EP 0 454 587 A1

La présente invention concerne de nouveaux dérivés doués de propriétés anti-cancéreuses appartenant à la famille des 2-styryl 4H-1-benzopyrane-4-ones, leur procédé de fabrication et les compositions pharmaceutiques qui les contiennent.

Des dérivés de la même famille sont connus, mais pour leurs propriétés anti-allergiques. Les brevets BE 885319, BE 855657, BE 869407 décrivent des dérivés de cette structure dont la position 3 est occupée par un alcoyle et la position 6 par un carboxyle (ou carboxylate) revendiqués en tant qu'inhibiteurs de manifestations allergiques.

Plus particulièrement, le brevet EP 237166 décrit les propriétés cytotoxiques d'une 2-styryl chromone, l'hormothamnione, isolée d'une algue.

Les besoins de la thérapeutique exigent le développement constant de nouveaux anti-cancéreux dans le but d'obtenir des molécules à la fois plus actives mais également moins toxiques, et si possible, agissant selon des mécanismes originaux.

Les composés de la présente invention se distinguent par leurs propriétés différenciatrices cellulaires et non plus uniquement cytotoxiques, indiquant une utilisation thérapeutique possible en tant qu'anti-cancéreux.

Plus particulièrement, la présente invention a pour objet des molécules de formule générale :

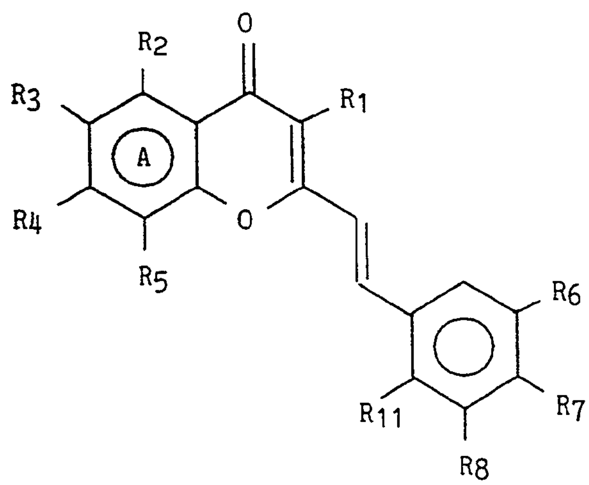

(I)

dans laquelle :

$R_1$ représente un atome d'hydrogène, un groupement alcoyle inférieur, hydroxyle ou alcoyloxyle inférieur,

$R_6$, $R_7$, $R_8$, $R_{11}$ identiques ou différents représentent indépendamment l'un de l'autre un atome d'halogène, un groupement hydroxyle, alcoyloxyle inférieur, thio, alcoylthio inférieur, sulfonyle, alcoylsulfonyle inférieur, ou un groupement de formule (G) :

D-(A)m-(O-CO)p-(B)n **(G)**

avec n, m, p, identiques ou différents, égaux à 0 ou 1, étant entendu que si p = 0, alors m = 0 ;

ou bien $R_8$ et $R_{11}$ forment avec le cycle aromatique qui les porte un groupement naphtyl,

A et B identiques ou différents représentent un groupe alcoyle inférieur linéaire ou ramifié ou alcényle inférieur linéaire ou ramifié ;

D représente un atome d'hydrogène ou un groupement de formule :

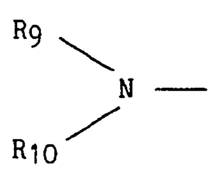

avec $R_9$ et $R_{10}$ identiques ou différents, représentant indépendamment l'un de l'autre, un atome d'hydrogène, un groupement alcoyle inférieur, un groupement acyl alcoyl inférieur ou formant avec l'atome d'azote qui les porte un système hétérocyclique azoté mono ou bicyclique comprenant de 4 à 10 sommets parmi lesquels on peut trouver un ou deux hétéroatomes choisis parmi oxygène, soufre ou azote ;

$R_2$, $R_3$, $R_4$, $R_5$ identiques ou différents représentent un atome d'halogène, hydroxyle, alcoyloxy inférieur ou un

groupement de formule (G) tel que ci-dessus défini,

– étant entendu que :

. l'un au moins de $R_3$ ou $R_5$ représente un groupement de formule (G) différent de l'hydrogène,

. si $R_3$ représente un groupement de formule (G) avec n = 0 et p = 1, alors l'un au moins de $R_2$, $R_4$, $R_5$ est différent de l'hydrogène,

. si $R_5$ représente un groupement méthyle ou si $R_3$ représente un groupement alcoyle inférieur, alors l'un au moins des trois autres substituants du cycle aromatique A est différent de l'hydrogène,

ou bien $R_3$ et $R_4$ forment ensemble avec le cycle aromatique qui les porte un système bi ou tricyclique de 9 à 15 atomes, saturé ou non susceptible d'inclure dans un squelette carboné un ou plusieurs hétéroatomes choisis parmi l'oxygène, le soufre ou l'azote étant entendu que :

– lorsque $R_3$ et $R_4$ forment avec le cycle aromatique qui les porte un système benzofurannique ou benzopyrannique saturés ou non, $R_2$ et $R_5$ ne peuvent représenter un groupement hydroxyle ou alkoxy inférieur lorsque $R_6$, $R_7$, $R_8$ et $R_{11}$ simultanément représentent un atome d'hydrogène,

– et lorsque $R_3$ et $R_4$ forment avec le cycle aromatique qui les porte un système benzofurannique non saturé, $R_7$ ne peut représenter un groupement alkyle lorsque $R_6$, $R_8$ et $R_{11}$ représentent simultanément un atome d'hydrogène, R2 un groupement alkoxy et R5 un groupement alkoxy ou un atome d'hydrogène,

leurs isomères (la configuration autour de la double liaison peut-être E ou Z, préférentiellement E), énantiomères, diastéréoisomères ainsi que, lorsque la molécule comprend un groupement acide, leurs sels d'addition à une base pharmaceutiquement acceptable et lorsque la molécule contient un groupement basique, leurs sels d'addition à un acide pharmaceutiquement acceptable, étant entendu que par groupements alcoyle inférieur, alcényle inférieur et alcoyloxy inférieur on entend des groupements linéaires ou ramifiés comprenant de 1 à 6 atomes de carbone.

Parmi les bases susceptibles de salifier les composés de formule (I) dans lesquels R représente un groupement carboxyle, on peut citer à titre d'exemple, les hydroxydes de sodium, potassium, calcium ou d'aluminium, les carbonates de métaux alcalins ou alcalino-terreux, ou des bases organiques comme la triethylamine, la benzylamine, la diéthanolamine, la tert-butylamine, la dicyclohexylamine, l'arginine, etc ...

Parmi les acides susceptibles de salifier les composés de composés de formule (I), on peut citer à titre d'exemple non limitatif les acides chlorhydrique, sulfurique, tartrique, maléique, fumarique, oxalique, methanesulfonique, camphorique, etc ...

Parmi les composés de l'invention, on préfère ceux pour lesquels :

– $R_3$ représente un groupement de formule (G) avec p différent de 0,

– $R_5$ représente un groupement de formule (G) avec n et p différents de 0,

– $R_3$ et $R_4$ forment avec le cycle aromatique qui les porte un système benzofurannique.

La présente invention s'étend également aux procédés de préparation des dérivés de formule (I), caractérisé :

– ou bien (PROCEDE A), par le fait que l'on condense par chauffage à reflux du solvant un dérivé de formule (II),

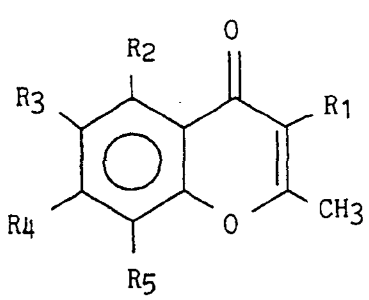

(II)

dans lequel $R_1$, $R_2$, $R_3$, $R_4$ et $R_5$ possèdent la même signification que dans la formule (I) ,

avec un aldéhyde benzoïque substitué (III) dans lequel $R_6$, $R_7$, $R_8$ et $R_{11}$ possèdent la même signification que dans le formule (I),

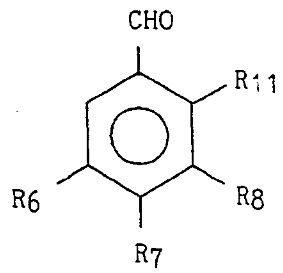

(III)

en présence d'un agent basique dans un solvant polaire, pour conduire après refroidissement et filtration du milieu réactionnel à un composé de formule (I) ;

– ou bien (PROCEDE B), par le fait que l'on traite deux molécules d'halogénure de triphénylalcoylphosphonium (ou triphénylalcoyloxy methylphosphonium) de formule (IV) :

$$R_1 - CH_2 - P^{\oplus} (C_6H_5)_3 X^{\ominus} \quad \textbf{(IV)}$$

dans laquelle $R_1$ représente un atome d'hydrogène , un alcoyle inférieur, un alcoyloxyle inférieur et $X^{\ominus}$ l'anion d'un hydracide, en suspension dans un solvant organique, préférentiellement le tétrahydrofurane anhydre, par une base forte, préférentiellement une solution de n-butyllithium dans l'hexane, qui est additionnée progressivement et sous agitation, la solution obtenue après un contact de plusieurs heures à température préférentiellement ambiante étant, à son tour, traitée sous atmosphère inerte par une molécule de 2-hydroxy benzoate d'alcoyle de formule (V) :

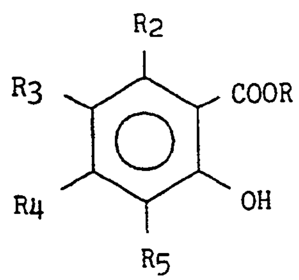

(V)

dans lequel $R_2$, $R_3$, $R_4$ et $R_5$ possèdent la même signification que dans la formule (I) et R représente un alcoyle inférieur ,

en solution dans le solvant précédemment choisi, le milieu obtenu étant ensuite chauffé à une température préférentiellement comprise entre 50°C et 60°C pour obtenir après filtration du milieu réactionnel et élimination sous vide des solvants un dérivé de formule (VI) :

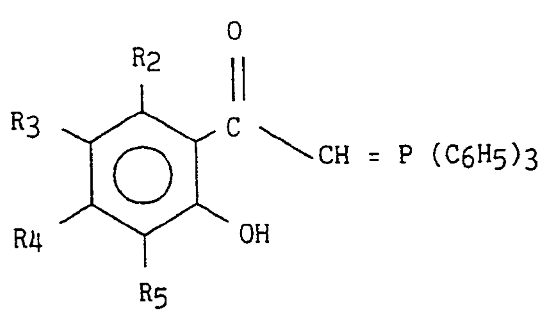

(VI)

dans lequel $R_2$, $R_3$, $R_4$ et $R_5$ possèdent la même signification que précédemment,

qui, placé dans la pyridine anhydre et sous atmosphère inerte, est additionné du chlorure de l'acide cinnamique de formule (VII) :

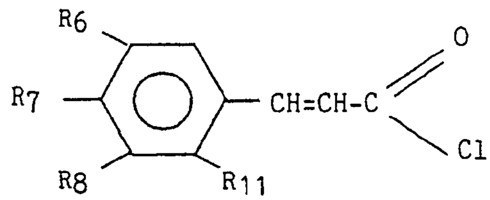

(VII)

dans lequel $R_6$, $R_7$, $R_8$ et $R_{11}$ possèdent la même signification que dans la formule (I), et, chauffé à une température préférentielle de 60°C pendant une journée environ pour conduire, après élimination du solvant, reprise par un solvant organique approprié et extractions successives en milieu alcalin et enfin élimination des solvants, à un composé de formule (I) qui,

quel que soit le procédé selon lequel il a été obtenu, est, si on le désire,

- soit, lorsque $R_1$, $R_2$, $R_3$, $R_4$, $R_5$, $R_6$, $R_7$, $R_8$ ou $R_{11}$ représentent (ou comprennent) un groupement carboxylique, salifié par une base pharmaceutiquement acceptable ou estérifié par un alcool pharmaceutiquement acceptable ou lorsque les mêmes restes comportent un groupement basique, salifié par un acide pharmaceutiquement acceptable,
- soit, séparé en ses stéréoisomères, diastéréoisomères, énantiomères par une technique de cristallisation ou de chromatographie, puis, si on le désire, lorsque $R_2$, $R_3$, $R_4$, $R_5$, $R_6$, $R_7$, $R_8$ ou $R_{11}$ représentent (ou comprennent) un groupement carboxylique, procéder aux mêmes opérations que celles décrites précédemment.

Le procédé A sera préférentiellement utilisé pour l'obtention des : 2-styryl 5H-furo (3,2- g) -1- benzopyrane-5-ones (IX) à partir des composés (VIII) :

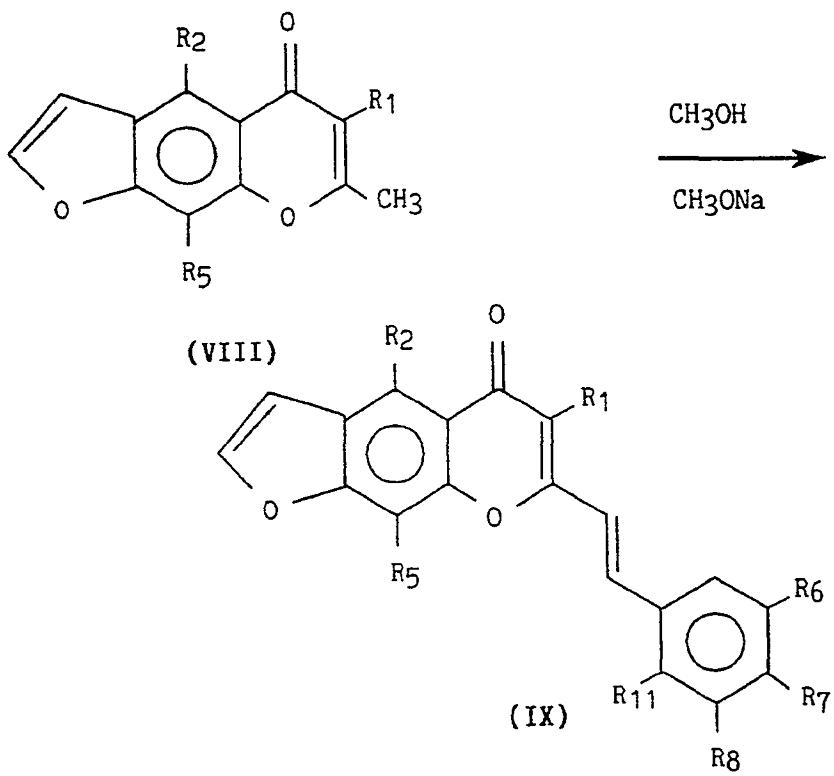


(VIII)

(IX)

les rendements variant de 65 à 70 %,

et des (-2-styryl oxo-4 4H-1-benzopyran-6-yl) carboxylates d'alcoyles (XI) à partir des composés (X) :

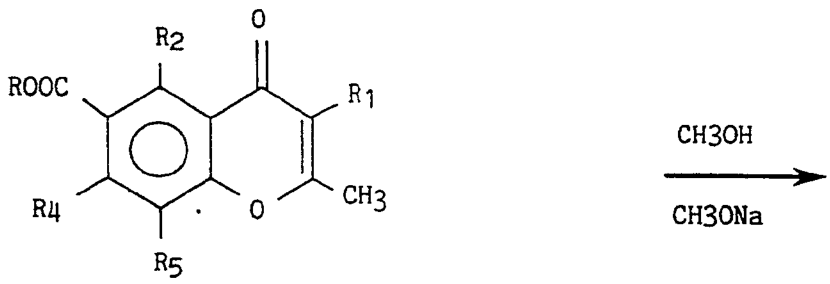


(X)

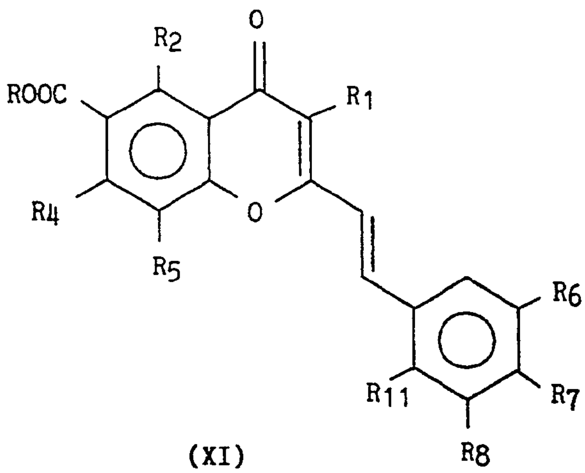

(XI)

dans lesguels R représente un atome d'hydrogène ou un groupement alcoyle inférieur,

Les composés de formule (I) possèdent d'intéressantes propriétés pharmacologiques. Ils inhibent, de façon plus importante que l'acide rétinoïque, la croissance de la lignée L1210, et fait particulièrement intéressant pour une utilisation en thérapeutique, induisent une différenciation cellulaire de la lignée HL60, sans entrainer de toxicité hypervitaminique A, ce qui est le cas de la référence : l'acide rétinoïque. In vivo chez la Souris, certains composés se révèlent actifs sur l'adéno-carcinome C38.

Les composés décrits dans la présente invention trouvent donc une utilisation en thérapeutique en tant qu'agents antitumoraux, pour le traitement ou la prophylaxie des néoplasmes bénins ou malins dont le carcinome du colon, ainsi que dans les indications classiques de cette classe thérapeutique tels que les désordres cutanés (acné, psoriasis) ainsi que les troubles dégénératifs et/ou inflammatoires des muqueuses.

La présente invention a également pour objet les compositions pharmaceutiques contenant les produits de formule (I), ou un de leurs sels d'addition à une base ou un acide pharmaceutiquement acceptable, lorsque les restes $R_1$ à $R_8$ représentent un groupement salifiable seul ou en combinaison avec un ou plusieurs excipients ou véhicules inertes, non toxiques, pharmaceutiquement acceptable.

Parmi les compositions pharmaceutiques selon l'invention, il sera cité plus particulièrement celles qui conviennent pour l'administration orale, parentérale, nasale, rectale, perlinguale, oculaire ou respiratoire, et notamment les comprimés simples ou dragéifiés, les comprimés sublinguaux, les sachets, les paquets, les gélules, les glossettes, les tablettes, les suppositoires, les crèmes, les pommades, les gels dermiques, les préparations injectables ou buvables, les aérosols, les gouttes oculaires ou nasales, etc...

La posologie utile varie selon l'âge et le poids du patient, la voie d'administration, la nature de l'indication thérapeutique et des traitements éventuellement associés et s'échelonne entre 0,1 et 200 mg par jour.

Les exemples suivants illustrent l'invention, mais ne la limitent en aucune façon.

Les spectres infra-rouge sont réalisés soit en film, soit en pastille de bromure de potassium (1 %) selon l'état liquide ou solide des produits. Les spectres de résonance magnétique nucléaire, sauf indication contraire, sont réalisées dans le chloroforme deutérié ou dans la diméthylsulfoxyde hexadeutérié avec un appareil à 100 MHz en utilisant le tétramethylsilane comme référence interne, les déplacements étant exprimés en ppm.

Les préparations ne font pas partie de l'invention mais en illustrent des modes de réalisation.

**PREPARATION 1 : 4 -METHOXY - 7 - STYRYL - 5 H - FURO (3,2 - g) BENZOPYRAN - 5 - ONE**

A 2 g (8,7 mmol) de visnagine solubilisés à chaud dans le minimum de méthanol anhydre (150 ml) sont ajoutés, sous agitation et à l'abri de l'humidité, une solution de méthylate de sodium (34,8 mmol ; 0,80 g de Na dans 20 ml de méthanol), puis 1,32 ml (13,05 mmol) de benzaldéhyde. Après 24 h de reflux, le milieu réactionnel est filtré et le précipité extrait plusieurs fois par l'oxyde diéthyle, puis séché ; 1,6 g de cristaux beiges est obtenu.

Rdt : 56 % F : 169 °C (oxyde de diéthyle)

Analyses élémentaires :

Calculé : C = 75,46 %, H = 4,43 % ;

Trouvé : C = 74,61 %, H = 4,81 %.

IR (KBr 1 %, cm-1) : 2820, 1650, 1615, 1460, 1150

RMN du 1H ($CDCl_3$, δ ppm) : 4,20 (s, 3H, $OCH_3$-4) ; 6,20 (s, 1H, H6); 6,65-6,80 (d, $J_{\alpha-\beta}$ = 16 Hz, 1H, $H_\alpha$ ) ; 7,05-7,60 (m, 9H, H2, H4, Har et $H_\beta$)

SDM (m/z) : 318, 289, 215, 190,161,128

**EXEMPLE 1 :**

**5,7 - DIMETHOXY - 4 - OXO - 2 - STYRYL - 4H - 1 - BENZOPYRANE - 6 - CARBOXYLATE DE METHYLE**

**STADE A :** 7 - HYDROXY- 5 - METHOXY - 2 - METHYL - 4 - OXO - 4H - 1 - BENZOPYRANE - 6 CARBOXYLATE DE METHYLE

A 2 g (8,5 mmol) de 6-formyl-7-hydroxy-5-méthoxy -2-méthyl - 4H - benzopyrane -4-one (A. SCHONBERG, N. BADRAN et N.A. STARKONSKY, J. Am. Chem. Soc., 1953, 75, 4992) en suspension dans 105 ml de méthanol acidifié par 2,6 ml d'acide acétique, sont ajoutés 2,6 g (53 mmol) de cyanure de sodium, puis 18,50 g (223 mmol , 25 eq) de dioxyde de manganèse activé. Après 4 h d'agitation à température ambiante, le milieu réactionnel dilué par 200 ml d'acétate d'éthyle est filtré pour éliminer l'insoluble ($MnO_2$ en excès) et les solvants sont évaporés. Le résidu poudreux est repris par l'eau et filtré. 1,80 g d'une poudre beige est obtenu.
Rdt : 80 % F : 200 °C

**STADE B :** 5,7 - DIMETHOXY- 2 -METHYL - 4 - OXO - 4H - 1 - BENZOPYRANE - 6 - CARBOXYLATE DE METHYLE

A l'abri de l'humidité et à 60°C, 0,304 g (7,6 mmol) d'hydrure de sodium (dispersion à 60 %) est ajouté, lentement et sous agitation, à 2 g (7,6 mmol)de 7-hydroxy-5-méthoxy - 2 - méthyl - 4- oxo - 4H-1-benzopyrane-6-carboxylate de méthyle en solution dans 25 ml de DMF anhydre. Après un contact d'1h30, 0,95 ml (15,2mmol) d'iodométhane est ajouté au milieu réactionnel et l'agitation poursuivie pendant 2 h. Après décomposition de l'excès d'hydrure de sodium par addition de 6 ml de méthanol, le milieu réactionnel est dilué avec de l'oxyde diéthyle, puis versé sur 100 ml d'eau. Le précipité formé est filtré, puis lavé plusieurs fois par de l'oxyde de diéthyle. Une purification par chromatographie sur colonne de gel de silice (éluant : $CH_2Cl_2$ / $CH_3OH$ : 99/1) du résidu poudreux conduit à 2,0 g de cristaux blancs.

Rdt : 95 % F : 188 °C

Analyses élémentaires : $C_{14}H_{14}O_6$, 0,5 $H_2O$ Mr : 287,27

Calculé : C = 58,53 %, H = 5,26 % ;

Trouvé : C = 58,53 %, H = 5,22 %.

**STADE C :** 5,7 - DIMETHOXY - 4 - OXO - 2 - STYRYL - 4H - 1 - BENZOPYRANE - 6- CARBOXYLATE DE METHYLE

A 2 g (7,2 mmol) de 5,7 - diméthoxy - 2- méthyl - 4- oxo - 4H - 1 - benzopyrane - 6 - carboxylate de méthyle solubilisés à chaud dans 250 ml de méthanol anhydre, sont ajoutés, sous agitation et à l'abri de l'humidité, une solution de méthylate de sodium (28,8 mmol, 0,662 g de Na dans 20ml de $CH_3OH$), puis 1,15 g (10,8 mmol) de benzaldéhyde. Après 24 h de reflux, le milieu réactionnel est concentré (élimination d'environ 200 ml de méthanol), puis refroidi. Le précipité est filtré et extrait plusieurs fois par de l'oxyde de diéthyle ; 2,2 g de cristaux blancs sont obtenus.

Rdt : 80 % F : 172 °C (oxyde de diéthyle)

| | | | |
|---|---|---|---|
| Analyses élémentaires | : $C_{21}H_{18}O_6$, | | Mr : 366,37 |
| Calculé | : C = 68,84 %, | H = 4,95 % ; | |
| Trouvé | : C = 68,97 %, | H = 5,31 %. | |

| | |
|---|---|
| IR (KBr 1 %, $cm^{-1}$) | : 1735, 1645, 1610, 1590, 750 et 690 |
| RMN du $^1H$ ($CDCl_3$, δ ppm) | : 3,85 (s, 3H, $COOCH_3$) ; 3,90 (s, 3H, $OCH_3$-7); 3,95 (s, 3H, $OCH_3$-5) ; 6,20 (s, 1H, H3) ; 6,70 (d, $J_{\alpha-\beta}$ = 16 Hz, 1H, <u>CH</u> = CH-$C_6H_5$) ; 7,50 (d, $J_{\beta-\alpha}$= 16 Hz, 1H, CH = <u>CH</u>-$C_6H_5$) ; 7,40-7,60 (m, 6H, Har) |
| SDM (m/z) | : 366, 351, 334, 305 , 277, 128 |

**EXEMPLE 2 : 5,7 - DIMETHOXY - 4 - OXO - 2 - (3,4 - DIMETHOXY STYRYL) - 4H - 1 - BENZOPYRANE - 6 - CARBOXYLATE DE METHYLE**

Selon le protocole décrit pour le stade C de l'exemple 1.

Rdt : 81 % F : 176 °C (oxyde de diéthyle)

| | | | |
|---|---|---|---|
| Analyses élémentaires | : $C_{23}H_{22}O_8$, 0,5 $H_2O$ ; | | Mr : 435,42 |
| Calculé | : C = 63,44 %, | H = 5,32 % ; | |
| Trouvé | : C = 63,42 %, | H = 5,39 %. | |

| | |
|---|---|
| IR (KBr 1 %, $cm^{-1}$) | : 1730, 1640, 1620, 1600, 840 et 800. |
| RMN du $^1H$ ($CDCl_3$, δ ppm) | : 3,80 (s, 3H, $OCH_3$-3') ; 3,85 (s, 3H, $COOCH_3$) ; 3,90 (s, 6H, $OCH_3$-7 et 4') ; 3,95 (s, 3H, $OCH_3$-5) ; 6,15 (s, 1H, H3) ; 6,50 (d, $J_{\alpha-\beta}$ = 16 Hz, 1H, <u>CH</u> = CH-Ar) ; 6,75-6,90 (m, 4H, Har) 7,05 (d, $J_{\beta-\alpha}$= 16 Hz, 1H, CH = <u>CH</u>-Ar) ; |
| SDM (m/z) | : 426, 411, 394, 365 , 337, 188 |

**EXEMPLE 3 : 5,7 - DIMETHOXY - 4 - OXO - 2 - (3,4,5 - TRIMETHOXY STYRYL) - 4H - 1 - BENZOPYRANE - 6 - CARBOXYLATE DE METHYLE**

Selon le protocole décrit pour le stade C de l'exemple 1 (sauf le temps de chauffage : 36 h au reflux du méthanol).

Rdt : 65 % F : 171 °C (oxyde de diéthyle)

| | | | |
|---|---|---|---|
| Analyses élémentaires | : $C_{24}H_{24}O_9$, 1 $H_2O$ ; | | Mr : 474,45 |
| Calculé | : C = 60,75 %, | H = 5,22 % ; | |
| Trouvé | : C = 60,26 %, | H = 5,58 %. | |

IR (KBr 1 %, $cm^{-1}$) : 1735, 1640, 1610, 1575 et 850

RMN du $^1H$ ($CDCl_3$, δ ppm) : 3,80 (s, 6H, $OCH_3$-3' et 5') ; 3,85 (s, 3H, $COOCH_3$) ; 3,90 (s, 6H, $OCH_3$-7 et 4') ; 3,95 (s, 3H, $OCH_3$-5) ; 6,20 (s, 1H, H3) ;
6,60 (d, $J_{\alpha-\beta}$ = 16 Hz, 1H, <u>CH</u> = CH-Ar) ;
6,70-6,80 (m, 3H) ;
7,40 (d, $J_{\beta-\alpha}$= 16 Hz, 1H, CH = <u>CH</u>-Ar).

SDM (m/z) : 456, 441, 424, 395 , 367, 218

**EXEMPLE 4 : 5,7 - DIMETHOXY - 2 - (4 - METHOXYCARBONYL - STYRYL) - 4H - 1 BENZOPYRANE - 6 - CARBOXYLATE DE METHYLE**

Selon le protocole décrit pour le stade C de l'exemple 1 (sauf le temps de chauffage : 3 h au reflux du méthanol).

Rdt : 70 % F : 197 °C (oxyde de diéthyle)

| | | | |
|---|---|---|---|
| Analyses élémentaires | : $C_{23}H_{20}O_8$, | | Mr : 424,40 |
| Calculé | : C = 65,09 %, | H = 4,79 % ; | |
| Trouvé | : C = 64,93 %, | H = 4,94 %. | |

IR (KBr 1 %, $cm^{-1}$) : 1735, 1720, 1640, 1610, 1600, 780

RMN du $^1H$ ($CDCl_3$, δ ppm) : 3,85 (s, 3H, $COOCH_3$-6) ; 3,90 (s, 6H, $OCH_3$-7 et $COOCH_3$-4') ; 3,95 (s, 3H, $OCH_3$-5) ; 6,20 (s, 1H, H3) ;
6,75 (d, $J_{\alpha-\beta}$ = 16 Hz, 1H, <u>CH</u> = CH-Ar) ;

6,80 (s, 1H, H8) ;
7,60 (-d, $J_{\beta-\alpha}$= 16 Hz, 1H, CH = CH-Ar) ;
7,60-8,10 (m, 4H, Har)

SDM (m/z) : 424, 409, 392, 363 , 335

**EXEMPLE 5 : ACIDE 4 - [ (5,7 - DIMETHOXY - 6 - METHOXYCARBONYL - 4 - OXO - 4H - 1 - BENZOPYRAN - 2 - YL) - VIN - 1 - YL ] BENZOIQUE**

Selon le protocole décrit pour le stade C de l'exemple 1.

Rdt : 55 % F : 244 °C (éthanol)
Analyses élémentaires : $C_{22}H_{18}O_8$, $1H_2O$ Mr : 428,39
Calculé : C = 60,75 %, H = 5,52 % ;
Trouvé : C = 61,12 %, H = 5,45 %.

IR (KBr 1 %, cm-1) : 1735, 1700, 1640, 1610, 1580, 800
RMN du $^1H$ ($CDCl_3$, δ ppm) : 3,85 (s, 3H, $COOCH_3$-6) ; 3,90 (s, 3H, $OCH_3$-7); 3,95 (s, 3H, $OCH_3$-5) ; 6,35 (s, 1H, H3) ;
7,30 (d, $J_{\alpha-\beta}$ = 17 Hz, 1H, CH = CH-Ar) ;
7,75-8,05 (m, 5H, Har) ; 8,00 (d, $J_{\beta-\alpha}$= 17 Hz, 1H, CH = CH-Ar) ;
13,00 (s, 1H, COOH)

**EXEMPLE 6 : 4 - OXO - 2 - STYRYL - 5,7,8 - TRIMETHOXY - 4H - 1 - BENZOPYRANE - 6 - CARBOXYLATE DE METHYLE**

**STADE A :** 5,7 - DIMETHOXY- 7- HYDROXY - 2 - METHYL - 4 - OXO - 4H - 1 - BENZOPYRANE - 6- CARBOXYLATE DE METHYLE

A 2 g (7,5 mmol) de 5,8- diméthoxy - 6- formyl - 7- hydroxy - 2-méthyl - 4H - 1 benzopyran - 4 - one (R.B. GAMMILL et S.A. NASH, J. Org. Chem., 1986, 51, 3116) en suspension dans 75 ml de méthanol acidifié par 2 ml d'acide acétique, sont ajoutés 1,84 g (37,5 mmol) de cyanure de sodium, puis 13,04 g (161 mmol) de dioxyde de manganèse activé. Après 4 h d'agitation à température ambiante, le milieu réactionnel dilué par 100 ml d'acétate d'éthyle est filtré pour éliminer l'insoluble ($MnO_2$ en excès) et les solvants sont évaporés. Le résidu poudreux est repris par $H_2O$ et filtré. 1,103 g d'une poudre grisâtre est obtenu.
Rdt : 50 % F : 210 °C

**STADE B :** 2 - METHYL - 4 - OXO - 5,7,8 - TRIMETHOXY - 4H - 1 - BENZOPYRANE - 6- CARBOXYLATE DE METHYLE

A l'abri de l'humidité et à 60°C, 0,42 g (10,6 mmol) d'hydrure de sodium (dispersion à 60 %) est ajouté lentement et sous agitation à 3,10 g (10,6 mmol) de 5,8 - diméthoxy - 7 - hydroxy - 2 - méthyl - 4 - oxo - 4H - 1 - benzopyrane - 6 carboxylate de méthyle en solution dans 50 ml de diméthylformamide anhydre. Après un contact de 1 h 30, 1,30 ml (21,2 mmol) ; d'iodométhane est ajouté au milieu réactionnel et l'agitation poursuivie pendant 2 h. Après décomposition de l'excès d'hydrure de sodium par addition de méthanol, le milieu réactionnel dilué par l'oxyde de diéthyle est filtré. L'évaporation du filtrat conduit à une huile orangée qui est reprise par l'eau et extraite par 3 x 50 ml d'oxyde de diéthyle . Les phases organiques réunies sont lavées par une solution saturée de NaCl, séchées sur $Na_2SO_4$, puis les solvants éliminés. Une chromatographie sur colonne de gel de silice (70-230 mesh ; éluant : $CH_2Cl_2$ / $CH_3OH$ : 99/1) du résidu poudreux livre 2,5 g de cristaux orangés.
Rdt : 76 % F : 125 °C

**STADE C :** 4 - OXO - 2 - STYRYL - 5,7,8 - TRIMETHOXY - 4H - 1 - BENZOPYRANE - 6- CARBOXYLATE DE METHYLE

A 1 g (3,24 mmol) de 2-méthyl-4-oxo- 5,7,8 - triméthoxy - 4H- 1 - benzopyrane - 6-carboxylate de méthyle solubilisé à chaud dans 200 ml de méthanol anhydre, sont ajoutés, sous agitation et à l'abri de l'humidité, une solution de méthylate de sodium (6,48 mmol ; 0,15 g de Na° dans 10 ml de méthanol anhydre), puis 0,52 g (4,85 mmol) de benzaldéhyde. Après 24 h de reflux, le milieu réactionnel est concentré ; le résidu repris par 150 ml d'eau est acidifié par une solution d'HCl N et extrait par 3 x 50 ml d'oxyde de diéthyle. L'élimination des solvants conduit à une huile orangée, qui, après purification par chromatographie sur colonne de gel de silice (70-230 mesh ; éluant : oxyde de diisopropyle), livre 0,250 g de cristaux orangés.

| | | | |
|---|---|---|---|
| Rdt : 40 % | F : 164 °C | | |
| Analyses élémentaires | : $C_{22}H_{20}O_7$, $1H_2O$ | | Mr : 414,39 |
| Calculé | : C = 63,76 %, | H = 5,35 % ; | |
| Trouvé | : C = 63,23 %, | H = 5,65 %. | |

IR (KBr 1 %, $cm^{-1}$) : 1740, 1635, 1620, 1600, 750 et 690

RMN du $^1H$ ($CDCl_3$, δ ppm) : 3,75 (s, 3H, $OCH_3$-8) ; 3,85 (s, 3H, $COOCH_3$-6); 3,90 (s, 3H, $OCH_3$-7) ; 4,00 (s, 3H,$OCH_3$-5) ;
6,75 (s, 1H H3) ;
7,40-7,55 (m, 6H, Har et H vinyl) ;
8,00 (d, $J_{\beta-\alpha}$= 17 Hz, 1H, CH = <u>CH</u>-$C_6H_5$)

**EXEMPLE 7 : 4-METHOXY-7-(3,4-DIMÉTHOXYSTYRYL)-5H-FURO(3,2-g) BENZOPYRAN - 5 - ONE**

Selon le protocole décrit pour la préparation 1.

| | | | |
|---|---|---|---|
| Rdt : 84 % | F : 182 °C (oxyde de diéthyle) | | |
| Analyses élémentaires | : $C_{22}H_{18}O_6$ | Mr = 396,38 | |
| Calculé | : C = 66,66 %, | H = 5,08 % ; | |
| Trouvé | : C = 66,49 %, | H = 4,84 %. | |

IR (KBr 1 %, $cm^{-1}$) : 2820, 1645, 1620, 1590, 850 et 820.

RMN du $^1H$ ($CDCl_3$, δ ppm) : 3,90 et 3,95 (s, 6H, $OCH_3$-3' et 4') ; 4,20 (s, 3H, $OCH_3$-4) ; 6,15 (s, 1H, H6);
6,55(d, $J_{\alpha-\beta}$ = 16 Hz, 1H, <u>CH</u>=CH-Ar ) ;
6,90-7,40 (m, 6H H vinyl, Har, H3 et 9) ;
7,60 (d, $J_{H2-H3}$ = 2 Hz, 1H, H3)

SDM (m/z) : 378, 349, 215, 190,188,161

**EXEMPLE 8 : 4 -METHOXY - 7 - (3,4,5 - TRIMETHOXY) - 5 H-FURO (3,2- g) BENZOPYRAN - 5 - ONE**

Selon le protocole décrit pour la préparation 1 à l'exception du temps de chauffage ramené à 30 minutes.

| | | | |
|---|---|---|---|
| Rdt : 70 % | | F : | 204 °C (oxyde de diéthyle) |
| Analyses élémentaires | : $C_{23}H_{20}O_7$ | Mr = | 408,41 |
| Calculé | : C = 67,66 %, | | H = 4,93 % ; |
| Trouvé | : C = 67,50 %, | | H = 5,04 %. |

IR (KBr 1 %, $cm^{-1}$) : 2820,1645, 1620, 1580, 840.

RMN du $^1H$ ($CDCl_3$, δ ppm) : 3,90 et 3,95 (s, 9H, $OCH_3$-3',4',5') ; 4,20 (s, 3H, $OCH_3$-4); 6,20 (s, 1H, H6) ; 6,65 (d, $J_{\alpha-\beta}$ = 16 Hz, 1H, $H_\alpha$ ) ; 6,55-6,80 (m, 2H, H2' et 6') ; 7,05 (d, $J_{H3-H2}$ = 2 Hz, 1H, H3 ), 7,40 (s, 1H, H9) ; 7,50 (d, $J_{\beta-\alpha}$ = 16 Hz, 1H, $H_\beta$) ; 7,60 (d, $J_{H2-H3}$ = 2 Hz, 1H, H2 )

SDM (m/z) : 408, 379, 218, 190,161

**EXEMPLE 9 : 4 - (2 - (4 -METHOXY - 5 - OXO - 5 H-FURO (3,2-G) BENZOPYRAN - 7 - YL) VIN - 1 - YL) BENZOATE DE METHYLE**

Selon le protocole décrit pour la préparation 1 à l'exception du temps de chauffage ramené à 30 minutes.

| | | | |
|---|---|---|---|
| Rdt : 98 % | | F : | 198 °C (oxyde de diéthyle) |
| Analyses élémentaires | : $C_{22}H_{16}O_6$ | Mr = | 376,37 |
| Calculé | : C = 70,21 %, | | H = 4,28 % ; |
| Trouvé | : C = 69,62 %, | | H = 4,57 %. |

IR (KBr 1 %, $cm^{-1}$) : 1730, 1650, 1610, 1580, 770.

RMN du $^1H$ (CD $Cl_3$, δ ppm) : 3,95 (s, 3H, $COOCH_3$) ; 4,20 (s, 3H, $OCH_3$); 6,25 (s, 1H, H6) ; 6,80 (d, $J_{\alpha-\beta}$ = 16 Hz, 1H, $H_\alpha$) ; 7,05 (d, $J_{H3-H2}$ = 2 Hz, 1H, H3) 7,40 (d, $J_{\beta-\alpha}$ = 16 Hz, 1H, $H_\beta$) ; 7,35-7,65 (m, 2H, H2 et H9) ; 7,60-8,10 (m, 4H, H2',3',5' et 6').

SDM (m/z) : 376, 347, 215, 161

**PREPARATION 2 : 4,9 - DIMETHOXY - 7 - STYRYL - 5 H-FURO (3,2-g) BENZOPYRAN - 5 - ONE**

A une solution limpide de 2,60 g (10 mmol) de khelline (solubilisée à chaud) et de 1,060 g (10 mmol) de

benzaldehyde est ajoutée goutte à goutte une solution d'éthylate de sodium (230 mg de Na dans 10 ml d'éthanol). Après 30 minutes à température ambiante, le produit précipite dans le milieu réactionnel. L'agitation est poursuivie 12 h. Les cristaux sont ensuite filtrés puis lavés à l'éthanol.

| | | | |
|---|---|---|---|
| Rdt : 72 % | | F : 192 °C (oxyde de diéthyle) | |
| Analyses élémentaires | : $C_{21}H_{16}O_5$ | Mr = 348,36 | |
| Calculé | : C = 72,40 %, | H = 4,63 % ; | |
| Trouvé | : C = 71,98 %, | H = 4,91 %. | |

IR (KBr 1 %, $cm^{-1}$) : 1640, 1615,1590, 750, 690.

RMN du $^1H$ ($CDCl_3$, δ ppm) : 4,10 (s, 3H, $OCH_3$-9) ; 4,25 (s, 3H, $OCH_3$-4); 6,20 (s, 1H, H6) ; 6,75 (d, $J_{\alpha-\beta}$ = 16 Hz, 1H, $H_\alpha$) ; 7,00 (d, $J_{H3-H2}$ = 2 Hz, 1H, H3 ), 7,25-7,70 (m, 6H, $H_\beta$ et Har) ; 7,65 (d, $J_{H2-H3}$ = 2 Hz, 1H, H2).

**EXEMPLE 10 : ACIDE (2- STYRYLCHROMON - 8 - YL) - ACETIQUE**

**STADE A :** 2,8 - DIMETHYLCHROMONE

A 40 g (0,097 mol) de (2-hydroxy-3-méthylbenzoyl)-méthylènetriphényl-phosphorane en solution dans 250 ml de toluène anhydre, sont ajoutés 19 ml (0,17 mol) d'anhydride acétique et 17 ml (0,21 mol) de pyridine. Le milieu réactionnel est chauffé au reflux 5 h, puis, après refroidissement filtré pour éliminer le sel de pyridinium formé. Le filtrat est lavé par une solution aqueuse de carbonate de sodium à 15 % et les solvants sont éliminés sous vide. La chromatographie du résidu poudreux sur colonne de gel de silice (70-230 mesh ; éluant : $CH_2Cl_2/CH_3OH$ : 99/1) livre 9,5 g de cristaux blancs.

| | | |
|---|---|---|
| Rdt : 56 % | | F : 114 °C |
| Analyses élémentaires | : $C_{11}H_{10}O_2$ | Mr = 174,20 |

IR (KBr 1 %, $cm^{-1}$) : 1640, 1600.

RMN du $^1H$ ($CDCl_3$, δ ppm) : 2,40 (s, 3H, $CH_3$-8) ; 2,50 (s, 3H, $CH_3$-2); 6,20 (s, 1H, H3) ; 7,25 (t, $J_{6-5}$ =$J_{6-7}$ = 8,0 Hz, 1H, H6) ; 7,50 (dd, $J_{7-6}$ et $J_{7-5}$ =2 Hz, 1H, H7 ), 8,00 (dd, $J_{5-6}$ =8,00 Hz et $J_{5-7}$ = 2Hz, 1H, H5).

**STADE B : 8 - BROMETHYL - 2 - METHYLCHROMONE**

Une solution de 3 g (17 mmol) de 2,8-diméthyl-chromone dans 50 ml de tétrachlorure de carbone additionnée de 1,5 mg de peroxyde de benzoyle et de 3,0 g (17 mmol) de N-bromosuccinimide est chauffée au reflux pendant 6h sous agitation et sous irradiation UV (lampe Philips 500 W). Le milieu réactionnel refroidi est filtré pour éliminer le succinimide surnageant et le solvant évaporé. La recristallisation dans le cyclohexane de la poudre livre 3,45 g de cristaux.

Rdt : 80 % F : 100 °C

Analyses élémentaires : $C_{11}H_9O_2Br$ Mr = 253,10

IR (KBr 1 %, $cm^{-1}$) : 1640, 1600, 750.

RMN du $^1H$ ($CDCl_3$, δ ppm) : 2,45 (s, 3H, $CH_3$-2) ; 4,70 (s, 2H, $CH_2$ Br); 6,20 (s, 1H, H3) ; 7,30 (t, 1H, $J_{6-5}$ =$J_{6-7}$ = 7 Hz, H6) ; 7,95 (dd, $J_{7-6}$ et $J_{7-5}$ =2 Hz, 1H, H7 ), 8,20 (dd, 1H, $J_{5-6}$ = 7 Hz et $J_{5-7}$ = 2Hz, H5).

**STADE C :** 8 - CYANOMETHYL - 2 - METHYLCHROMONE

A 5,9 g (23 mmol) de 8-bromométhyl-2 méthyl-chromone en solution dans 200 ml de dichlorométhane anhydre, sont ajoutés 5,45 g (35 mmol) de cyanure de tétraéthylammonium. Après une nuit de contact à température ambiante, le solvant est évaporé sous vide et le résidu repris par l'oxyde de diéthyle puis filtré. L'évaporation du filtrat et la purification par chromatographie sur colonne de gel de silice (70-230 mesh ; éluant : $CH_2Cl_2$) livrent 2,40 g de cristaux blancs.

Rdt : 52 % F : 138 °C

Analyses élémentaires : $C_{12}H_9O_2N$ Mr = 199,21

IR (KBr 1 %, $cm^{-1}$) : 2220, 1650, 1600.

RMN du $^1H$ ($CDCl_3$, δ ppm) : 2,45 (s, 3H, $CH_3$-2) ; 3,95 (s, 2H, $CH_2$ CN); 6,20 (s, 1H, H3) ; 7,50 (t, $J_{6-5}$ =$J_{6-7}$ = 9 Hz, 1H,H6) ; 7,80 (dd, $J_{7-6}$ et $J_{7-5}$ =2 Hz, 1H, H7 ), 8,20 (dd, $J_{5-6}$ = 9 Hz et $J_{5-7}$ = 2Hz, 1H, H5).

**STADE D :** ACIDE (2 - METHYLCHROMON - 8 - YL) - ACETIQUE

A une solution de 0,70 g (3,5 mmol) de 8-cyanométhyl-2-méthylchromone dans 8 ml d'acide acétique aqueux à 50 % (v/v), sont ajoutés goutte à goutte 4 ml d'acide sulfurique conc.. L'addition terminée, le milieu réactionnel est chauffé au reflux pendant 7 h. Le milieu réactionnel refroidi est versé sur 250 ml d'eau glacée et placé une nuit au réfrigérateur. Le précipité formé est solubilisé dans une solution préalablement chauffée à une température comprise entre 50 et 60°C d'hydrogénocarbonate de sodium à 5 %, puis filtré. Après acidification du filtrat par HCl concentré, l'insoluble obtenu est séparé par filtration, puis lavé par 3 x 20 ml d'eau et séché (0,73 g de cristaux blanchâtres).

Rdt : 96 % F : 228 °C

Analyses élémentaires : $C_{12}H_{10}O_4$ Mr = 218,21

IR (KBr 1 %, $cm^{-1}$) : 3400-3200, 1700, 1640.

RMN du $^1H$ (DMSO-d6, δ ppm) : 2,35 (s, 3H, $CH_3$-2) ; 3,90 (s, 2H, $CH_2$ COOH); 6,25 (s, 1H, H3) ; 7,40 (t, $J_{6-5}$ = 9Hz et $J_{6-7}$ = 9 Hz, 1H,H6) ; 7,60 (dd, $J_{7-6}$ et $J_{7-5}$ =2 Hz, 1H, H7 ), 8,00 (dd, $J_{5-6}$ = 9 Hz et $J_{5-7}$ = 2Hz, 1H, H5) ; 12,50 (s, 1H, OH).

**STADE E** : ACIDE (2 - STYRYL - METHYLCHROMON - 8 - YL) - ACETIQUE

A 0,73 g (3,34 mmol) d'acide (2-méthylchromon-8-yl)-acétique solubilisé à chaud dans le minimum de méthanol anhydre, sont ajoutés, sous agitation et à l'abri de l'humidité, une solution de méthylate de sodium (6,7 mmol ; 0,155 g de Na dans 20 ml de $CH_3OH$), puis 0,35 ml (3,4 mmol) de benzaldéhyde. Après 24 h de reflux, le milieu réactionnel est évaporé et le résidu solubilisé dans une solution aqueuse d'hydrogénocarbonate de sodium à 5 % préalablement chauffée à une température comprise entre 50 et 60 °C (la solution étant au besoin filtrée). Après acidification du filtrat par HCl N, l'insoluble est séparé par filtration, puis lavé par 3 x 20 ml d'eau et enfin séché. Un lavage de la poudre jaune obtenue, dans le chloroforme préalablement chauffé à 30-40 °C, livre 0,70 g de cristaux beiges.

Rdt : 55 % F : 202 °C
Analyses élémentaires : $C_{19}H_{14}O_4$ Mr = 315,31
Calculé : C = 72,37 %, H = 4,79 % ;
Trouvé : C = 72,40 %, H = 4,80 %.

IR (KBr 1 %, $cm^{-1}$) : 3200-2900, 1710, 1600 et 1570
RMN du $^1H$ (DMSO-d6, δ ppm) : 4,00 (s, 2H, $CH_2$ COOH) ; 6,45 (s, 1H, H3); 7,30-8,00 (m, 10H, Har et H vinyl) ; 11,50 (s, 1H, OH)
RMN du $^{13}$Hc (DMSO-d6, δ ppm) : 177(C4) ; 172 (C10 ); 161(C8a) ; 154 (C2) ; 136,5 (C7) ; 136 (C1') ; 135 (Cβ) ; 130 (C5a) ; 129 (C4') ; 128 (C3'-5') ; 126 (C2'-6') ; 124,9 (C5) ; 123,7 (C8) ; 123,5 (C6) ; 121 (Cα) ; 110 (C3) ; 37 (C9).

**EXEMPLE 11 : ACIDE (2- (3',4',5'-TRIMETHOXYSTYRYL)-CHROMON-8-YL)-ACETIQUE**

Préparé selon le protocole décrit au stade E de l'exemple 10.
Cristaux orangés.

Rdt : 62 % F : 212 °C
Analyses élémentaires : $C_{22}H_{20}O_7$, $H_2O$ Mr = 414,40
Calculé : C = 63,76 %, H = 5,35 % ;
Trouvé : C = 63,95%, H = 5,30 %.

| | | |
|---|---|---|
| IR (KBr 1 %, $cm^{-1}$) | : | 3200-2500, 2820, 1710, 1620, 1600 et 1580 |
| RMN du $^1H$ ($CDCl_3$, δ ppm) | : | 3,70 (s, 3H, $OCH_3$-4') ; 3,80 (s, 6H, $OCH_3$-3' et 5'); 4,00 (s, 2H, $CH_2$ COOH) ; 6,40 (s, 1H, H3) ; 7,00-7,70 (m, 7H, Har, H vinyl et H2', 6') ; 12,50 (s.e., 1H, OH) |
| RMN du $^{13}C$ (DMSO-d6, δ ppm) | : | 177(C4) ; 173 (C10 ); 162 (C8a) ; 154 (C2) ; 142 (C4'); 140 (C7) ; 136 (C1') ; 135 (Cβ); 132 (C5a) ; 131 (C3'-5') ; 128 (C8) ; 127 (C2'-6') ; 124 (C5) ; 122 (C6) ; 119 (Cα) ; 110 (C3) ; 61 ($OCH_3$-4') ; 57 ($OCH_3$-3' et 5') ; 39 (C9). |

**EXEMPLE 12 : ACIDE (2- (4-CARBOXYSTYRYL)-CHROMON-8-YL)- ACETIQUE**

Préparé selon le protocole décrit dans l'exemple 10.
Cristaux jaunes.

Rdt : 70 % F : > 305 °C

Analyses élémentaires : $C_{20}H_{14}O_6$, 0,5 $H_2O$ Mr = 359,32

Théorie : C = 66,85 %, H = 4,21 % ;

Trouvé : C = 66,98%, H = 4,15 %.

| | | |
|---|---|---|
| IR (KBr 1 %, $cm^{-1}$) | : | 3200-2500, 1730 et 1710, 1620, 1600 et 1570. |
| RMN du $^1H$ (CD $Cl_3$, δ ppm) | : | 4,00 (s, 2H, $CH_2$COOH) ; 6,50 (s, 1H, H3); 7,20-8,00 (m, 10H, Har, H vinyl et H2',3', 5', 6', OH) ; 13,00 (s, 1H, OH) |
| RMN du $^{13}C$ (DMSO-d6, δ ppm) | : | 177(C4) ; 172 (C10 ); 168 (C11) ; 160 (C8a); 154 (C2); 138 (C4) ; 136 (C7) ; 135,5 (C1'); 135 (Cβ) ; 131 (C5a) ; 129,5 (C3'-5') ; 127 (C2'-6') ; 125 (C5) ; 124 (C8) ; 123 (C6) ; 122 (Cα) ; 112 (C3) ; 39 (C9). |

**EXEMPLE 13 : ACIDE (7-MÉTHOXY-3-MÉTHYL-4-OXO-2-STYRYL-4H-1-BENZOPYRAN -8-YL) - ACÉTIQUE**

**STADE A :** 2,3-DIMÉTHYL-7-MÉTHOXY-4H-1-BENZOPYRAN-4-ONE

11,9 g (66,11 mmol) de 2-hydroxy-4-méthoxypropiophénone dans 24 ml d'anhydride acétique sont chauffés au reflux pendant 7 h en présence de 14,90 g (181,56 mmol) d'acétate de sodium fraîchement fondu Après élimination sous vide de l'excès d'anhydride acétique, le milieu réactionnel est versé sur 200 ml d'eau et extrait par 3 x 50 ml de dichlorométhane. Les phases organiques réunies sont lavées par une solution saturée de chlorure de sodium, puis séchées sur sulfate de sodium. L'évaporation des solvants et une purification par chromatographie sur colonne de gel de silice (éluant : $CH_2Cl_2$) livrent 7,45 g de cristaux beiges.

Rdt : 55%  F : 117°C

Analyses élémentaires : $C_{12}H_{12}O_3$ Mr = 204,22

IR (KBr 1%, cm-1) : 2820 ; 1630 ; 1600-1580 ; 1440.RMN du $^1H$ ($CDCl_3$ δppm) : 2,00 (s, 3H, $CH_3$-3) ; 2,40 (s, 3H, $CH_3$-2); 3,90 (s, 3H, $OCH_3$-7) ; 6,80 (d, $J_{8-6}$=2Hz, 1H, H8) ; 6,90 (dd, $J_{6-5}$=9Hz et $J_{6-8}$=2Hz, 1H, H6) ;8,10 (d, $J_{5-6}$=9Hz, 1H, H5).

**STADE B :** 8-CHLOROMÉTHYL-2,3-DIMÉTHYL-7-MÉTHOXY-4H-1-BENZOPYRAN-4-ONE

4,9 g (23,48 mmol) de 2,3-dimethyl-7-méthoxy-4H-1-benzopyran-4-one sont chauffés à 60°C pendant 5 h sous agitation, dans un mélange constitué par 35 ml d'acide chlorhydrique conc. et 1,2 g (40 mmol) de polyoxyméthylène. Après refroidissement, le milieu reactionnel est versé sur 100 ml d'eau glacée et placé une nuit à 5°C. Après filtration du milieu réactionnel, le précipité récupére est lavé par 3 x 50 ml d'eau puis séché. 5,15 g de cristaux blancs sont obtenus.

Rdt : 87% F : 171°C

Analyses élémentaires : $C_{13}H_{13}O_3Cl$ Mr = 253,60

IR (KBr 1%, cm-1) : 2820 ; 1640 ; 1600-1590 ; 1440 ; 790.

RMN du $^1H$ ($CDCl_3$ δppm) : 2,05 (s, 3H, $CH_3$-3) ; 2,45 (s, 3H, $CH_3$-2); 4,00 (s, 3H, $OCH_3$) ; 4,90 (s, 2H, $CH_2Cl$); 7,00 (d, $J_{6-5}$=9Hz, 1H, H6) ; 8,20 (d, $J_{5-6}$=9Hz, 1H, H5).

**STADE C :** (2,3-DIMÉTHYL-7-MÉTHOXY-4-OXO-4H-1-BENZOPYRAN-8-YL) ACÉTONITRILE

A une solution préalablement chauffée à une température comprise entre 60 et 70°C de 1,47 g (22,53 mmol) de cyanure de potassium dans 8 ml d'eau est ajoutée, goutte à goutte et sous agitation, une suspension de 3,27 g (12,96 mmol) de 8-chloromethyl-2,3-diméthyl-7-methoxy-4H-1-benzopyran-4-one dans 38 ml d'éthanol bouillant. L'addition terminée, le milieu réactionnel est chauffé au reflux pendant 4 h à 70°C. Après refroidissement du milieu réactionnel, addition d'eau glacée et filtration, les cristaux obtenus sont séchés et purifiés par chromatographie sur colonne de gel de silice (éluant : $CH_2Cl_2$). 2,45 g de cristaux beiges sont obtenus.

Rdt : 77% F : 185°C

Analyses élémentaires : $C_{14}H_{13}O_3N$ Mr = 243,26

IR (KBR 1%, CM-1) : 2820 ; 2220 ; 1640 ; 1600-1580 ; 1440 ; 1415.

RMN du $^1H$ ($CDCl_3$ δppm) : 2,05 (s, 3H, $CH_3$-3) ; 2,45 (s, 3H, $CH_3$-2) ; 3,90 (s, 2H, $CH_2CN$) ; 4,00 (s, 3H, $OCH_3$) ; 7,00 (d, $J_{6-5}$ =9Hz, 1H, H6) ; 8,15 (d, $J_{5-6}$=9Hz, 1H, H5).

**STADE D :** ACIDE (2,3-DIMÉTHYL-7-MÉTHOXY-4-OXO-4H-1-BENZOPYRAN-8-YL) ACÉTIQUE

A une solution de 3,9 g (16,05 mmol) de (2,3-diméthyl-7-méthoxy-4-oxo-4H-1-benzopyran-8-yl) acétonitrile dans 20 ml d'acide acétique aqueux à 50% (v/v), sont ajoutés, goutte à goutte, 20 ml d'acide sulfurique conc.. L'addition terminée, le milieu réactionnel est chauffé à 70°C pendant 4 h, puis après refroidissement, versé sur 150 ml d'eau glacée et placé une nuit à 5°C. Le précipité formé est recueilli par filtration, puis solubilisé dans une solution d'hydrogénocarbonate de sodium à 5% (préalablement chauffée à une température comprise entre 50 et 60°C). Après filtration et acidification du filtrat par l'acide chlorhydrique conc., l'insoluble obtenu est séparé par filtration, puis, lavé par 3 x 50 ml d'eau et séché. 3,70 g de cristaux blancs sont obtenus.

Rdt : 87% F : 211°C

Analyses élémentaires : $C_{14}H_{14}O_5$ Mr = 262,26

IR (KBr 1%, cm-1) : 3300-2900 ; 2820 ; 1740 ; 1630 ; 1610-1580 ; 1440.

RMN du $^1H$ (DMSO-d6, δppm) : 1,90 (s, 3H, $CH_3$-3);2,40 (s, 3H, $CH_3$-2) ; 3,70 (s, 2H, $CH_2COOH$) ; 3,90 (s, 3H, $OCH_3$) ; 7,20 (d, $J_{6-5}$=9Hz, 1H, H6) ;7,95 (d, $J_{5-6}$=9Hz, 1H, H5) ; 12,60 (s.e., 1H, OH).

**STADE E :** ACIDE (7-MÉTHOXY-3-MÉTHYL-4-OXO-2-STYRYL-4H-1-BENZOPYRAN-8-YL) ACÉTIQUE

A 1 g (3,82 mmol) d'acide (2,3-diméthyl-7-méthoxy-4-oxo-4H-1-benzopyran-8-yl) acétique solubilisé à chaud dans le minimum de méthanol anhydre sont ajoutés, sous agitation et à l'abri de l'humidité, une solution de méthylate de sodium (7,64 mmol, 0,176 g de Na dans 30 ml de $CH_3OH$), puis 0,4 ml (3,82 mmol) de benzaldéhyde. Après 7 h de reflux, le milieu réactionnel est versé sur 40 ml d'eau glacée acidifiée par addition d'HCl conc.. Les cristaux jaunes sont solubilisés dans une solution, préalablement chauffée à une température comprise entre 50 et 60°C, d'hydrogénocarbonate de sodium à 5%, puis filtrés. Après acidification du filtrat par HCl conc., l'insoluble est séparé par filtration, puis lavé par 3 x 20 ml d'eau et séché. Les cristaux jaunes obtenus sont, soumis ou non, selon le degré de pureté, à une estérification suivie d'une hydrolyse acide.

Rdt : 32% F : 282°C (éthanol)
Analyses élémentaires : $C_{21}H_{18}O_5$, 0,5 $H_2O$ Mr=359,37
Calculé : C=70,26%, H=5,05% ;
Trouvé : C=70,70%, H=5,00%.

IR (KBr 1%, $cm^{-1}$) : 3600-3200 ; 1720 ; 1630 ; 1590 ; 1570 ; 780 et 685.
RMN du $^1H$ (DMSO-d6, δppm) : 2,15 (s, 3H, $CH_3$-3) ; 3,90 (s, 2H, $CH_2$-COOH) ; 3,95 (s, 3H, $OCH_3$-7) ;7,25 (d, $J_{\alpha-\beta}$=16Hz, 1H, Hα) ; 7,75 (d, $J_{\beta-\alpha}$=16Hz, 1H, Hβ) ;7,20 - 8,00 (m, 7H, H5 et H6 et H2'-6') ; 12,5 (s, 1H, OH).
SDM (m/z) : 350 ; 349 ; 335 ; 305 ; 291 ; 273 ; 141.

Les stades F et G correspondent à la purification de l'acide (2-styryl-4H-1-benzopyran-8-yl) acétique.

**STADE F :** (7-MÉTHOXY-3-MÉTHYL-4-OXO-2-STYRYL-4H-1-BENZOPYRAN-8-YL) ACÉTATE D'ÉTHYLE

Une suspension de 1,80 mmol d'acide (7-méthoxy-3-méthyl-4-oxo-2-styryl-4H-1-benzopyran-8-yl) acétique dans 60 ml d'éthanol acidifié par 1,5 ml d'$H_2SO_4$ conc. est portée au reflux du solvant pendant 7 h. Après refroidissement, le milieu réactionnel est placé plusieurs heures à 5°C puis le précipité séparé par filtration est séché. Une chromatographie de la poudre sur colonne de gel de silice (éluant : $CH_2Cl_2/C_2H_5OH$ : 98/2), livre des cristaux jaunes analytiquement purs.

Rdt : 95 %
Analyses élémentaires : $C_{23}H_{22}O_5$, Mr= 378,45
Calculé : C= 72,99 %, H= 5,87 % ;
Trouvé : C= 72,76 %, H= 5,90 %.

IR (KBr , $cm^{-1}$) : 1730 ; 1620 ; 1610-1590 ; 780 et 690.
RMN du $^1H$ ($CDCl_3$, δppm) : 1,20(t, 3H, $COOCH_2CH_3$) ; 2,25 (s, 3H, $CH_3$-3) ; 3,95 (s, 2H, $CH_2$-COOEt) ; 4,00 (s, 3H, $OCH_3$-7) ; 4,20 (q, 2H, $COOCH_2$-$CH_3$) ; 7,00 (d, $J_{6-5}$=9Hz, 1H, H6) ; 7,20-7,65 (m, 7H, Hα Hβ, Har) ; 8,15 (d, $J_{5-6}$=9Hz, 1H, H5).
SDM (m/z) : 350, 349, 335, 305, 291, 273 ; 141.

**STADE G : ACIDE (7-MÉTHOXY-3-MÉTHYL-4-OXO-2-STYRYL-4H-1-BENZOPYRAN-8-YL) - ACÉTIQUE**

Une suspension de 0,21 mmol de l'ester précédent dans un mélange d'acide acétique (6 ml) et d'acide chlorhydrique conc. (3 ml) est portée au reflux du solvant pendant 6 h. Après refroidissement, 8 ml d'eau sont ajoutés au milieu réactionnel et les cristaux jaunes formés, séparés par filtration, sont séchés. Une recristallisation dans

l'éthanol livre des cristaux jaunes analytiquement purs.

**EXEMPLE 14 : ACIDE (2-(3,4-DIMÉTHOXYSTYRYL) -7-MÉTHOXY-3-MÉTHYL-4-OXO-4H-1-BEZOPYRAN-8-YL) ACÉTIQUE**

Selon le protocole décrit au(x) stade(s) E (éventuellement F et G) de l'exemple 13.

Rdt : 45% F : 240°C (éthanol)

Analyses élémentaires : $C_{23}H_{22}O_7$, Mr= 410,45

Calculé : C= 67,30 %, H= 5,41 % ;

Trouvé : C= 67,06 %, H= 5,50 % .

IR (KBr 1%, $cm^{-1}$) : 3600-3200, 1710, 1610, 1595, 1580, 830 et 780.

RMN du $^1H$ (DMSO-$d_6$, δppm) : 2,15 (s, 3H, $CH_3$-3) ; 3,90 (s, 2H, $CH_2COOH$); 3,85 et 3,95 (s, 9H, $OCH_3$-7, 3' et 4') ;7,05 (d, $J_{\alpha-\beta}$=18Hz, 1H, Hα) ; 7,30 (d, $J_{\beta-\alpha}$=18Hz, 1H, Hβ) ;7,00 - 7,45 (m, 4H, H2', 5', 6' et 6) ; 7,95 (d, $J_{5-6}$=9Hz, 1H, H5) ; 12,50 (s, 1H, OH).

**EXEMPLE 15 : ACIDE (2-(3, 4, 5-TRIMÉTHOXYSTYRYL) - 7 - METHOXY -3-MÉTHYL-4-OXO-4H-1-BENZOPYRAN-8-YL) - ACÉTIQUE**

Selon le protocole décrit au(x) stade(s) E (éventuellement F et G) de l'exemple 13.

Rdt : 42% F : 240°C (éthanol)

Analyses élémentaires : $C_{24}H_{24}O_8$, $2H_2O$ Mr=476,45

Calculé : C=60,50%, H=5,92% ;

Trouvé : C=60,40%, H=5,61%.

IR (KBr 1%, cm-1) : 3600-3200, 1720, 1610, 1580, 1570 et 800.

RMN du $^1H$ (DMSO-$d_6$, δppm) : 2,15(s,3H,$CH_3$-3) ; 3,70 (s, 2H, $CH_2COOH$); 3,85 et 3,95 (s, 12H, $OCH_3$-3', 4', 5' et 7) ; 7,05 - 7,50 (m, 6H, H5 et 6, H2' et 6', Hvinyl) ; 2,50 (s, 1H, OH).

**EXEMPLE 16 : ACIDE (2-(4-CARBOXYSTYRYL)- 7 - METHOXY-3-MÉTHYL-4-OXO-4H-1-BENZOPYRAN-8-YL) - ACÉTIQUE**

Selon le protocole décrit au(x) stade(s) E (éventuellement F et G) de l'exemple 13.

Rdt : 40% F > 305°C (éthanol)
Analyses élémentaires : $C_{22}H_{18}O_7$, Mr=394,39
Calculé : C=67,00 %, H=4,60 % ;
Trouvé : C=67,76 %, H=4,64 %.

IR (KBr 1%, cm-1) : 3600-3100, 1710, 1730, 1620, 1590 et 770.
RMN du $^1H$ (DMSO-d6, δppm) : 2,15 (s, 3H, $CH_3$-3) ; 3,90 (s, 2H, $CH_2COOH$) ; 3,95 (s, 3H, $OCH_3$-7) ;7,50 (m, 2H, H2' et 6') ;7,85 - 8,05 (m, 6H, H3' et 4', H5 et 6, Hvinyl), 12,75 (s, 2H, OH).

**PREPARATION 3 : 8-MÉTHYL-2-STYRYL-4H-1-BENZOPYRAN-4-ONE**

**STADE A : (2-HYDROXY-3-MÉTHYLBENZOYL)-MÉTHYLÈNETRIPHÉNYL-PHOSPHORANE**

A température ambiante, sous atmosphère d'azote, 31,25 ml (50 mmol) d'une solution de n-BuLi (1,6 M dans l'hexane) sont ajoutés, goutte à goutte et sous agitation, à 14,6 g (36 mmol) de sel de phosphonium en suspension dans 120 ml de tétrahydrofurane anhydre. Après un contact de 3 h, 3 g (18 mmol) de 2-hydroxy-3-méthylbenzoate de méthyle en solution dans 20 ml de tétrahydrofurane sont additionnés, goutte à goutte, au milieu réactionnel qui est alors chauffé 3 h à une température comprise entre 50 et 60°C, puis filtré. L'évaporation des solvants conduit à 5,9 g de cristaux jaunes recristallisés dans le méthanol.

Rdt : 80% F : 170°C
Analyses élémentaires : $C_{27}H_{23}O_2P$, Mr=410,46
Calculé : C=79,01%, H=5,65% ;
Trouvé : C=79,02%, H=5,61%.

IR (KBr 1%, cm-1) : 3400, 1590, 1540, 750, 720 et 690.
RMN du $^1H$ ($CDCl_3$, δppm) : 2,20 (s, 3H, $CH_3$-3) ; 6,65 (t, $J_{5-6}$=$J_{5-4}$=7Hz, 1H, H5) ; 7,15 (d., $J_{4-5}$=7Hz, 1H, H4) ;7,35 - 7,80(m,18H, $PPh_3$, H6, HC=P et OH).

**STADE B : 8-MÉTHYL-2-STYRYL-4H-1-BENZOPYRAN-4-ONE**

Sous atmosphère d'azote, 2 g (4,87 mmol) de (2-hydroxy-3-méthylbenzoyl)-méthylènetriphénylphosphorane et 2,43 g (14,6 mmol) de chlorure de cinnamoyle dans 200 ml de pyridine anhydre sont agités et chauffés à 60°C pendant 24 h. Après élimination de la pyridine, le résidu est repris par du dichlorométhane et extrait par une solution aqueuse de soude 1N, puis par une solution aqueuse saturée de NaCl jusqu'à neutralité. L'élimination du dichlorométhane conduit à 1 g d'une huile brunâtre qui, par cristallisation dans le méthanol, donne 0,6 g de fins cristaux jaunes.

Rdt : 60% F : 152°C
Analyses élémentaires : $C_8H_{14}O_2$, Mr=262
Calculé : C=82,44% , H=5,34% ;
Trouvé : C=82,22% , H=5,44%.

IR (KBr 1%, $cm^{-1}$) : 1640, 1610 et 1600.
RMN du $^1H$ ($CDCl_3$, δppm) : 2,60 (s, 3H, $CH_3$-8) ; 6,30 (s, 1H, H3) ; 6,80 (d, $J_{\alpha-\beta}$=16Hz, 1H, Hα) ; 7,20 - 7,65 (m, 8H, H6 et 7, Hβ, H2' à 6') ; 8,05 (dd, $J_{5-6}$=7,5 Hz, $J_{5-7}$=2,0 Hz, 1H, H5).
SDM (m/z) : 262, 261, 245, 155, 134, 128, 106, 77.

**EXEMPLE 17 : 3,8-DIMÉTHYL-2-STYRYL-4H-1-BENZOPYRAN-4-ONE**

**STADE A : (1-(2-HYDROXY-3-MÉTHYLBENZOYL)-ÉTHYLIDÈNE)-TRIPHÉNYL-PHOSPHORANE**

Selon le protocole décrit au stade A de la préparation 3.
Cristaux jaunes.

Rdt : 51% F : 150°C
Analyses élémentaires : $C_{28}H_{25}O_2P$, Mr=424,45
Calculé : C=79,23 %, H=5,94% ;
Trouvé : C=79,22%, H=5,90%.

IR et RMN du $^1H$ : Se reporter aux tableaux I et II.

**STADE B : 6,8 - DIMETHYL - 2 - STYRYL - 4H - 1 - BENZOPYRAN - 4 - ONE**

Selon le protocole décrit au stade B de la préparation 3 en remplaçant le (2-hydroxy-3-méthylbenzoyl)-méthylènetriphénylphosphorane par le (1-(2-hydroxy-3-méthylbenzoyl) éthylidène)-triphényl phosphorane. Cristaux orangés.

Rdt : 67% F : 136°C (Méthanol)
Analyses élémentaires : $C_{19}H_{16}O_2$, Mr=276,33
Calculé : C=82,58 %, H=5,83% ;
Trouvé : C=82,43%, H=5,74%.

IR et RMN du $^1H$ : Se reporter aux tableaux III et IV.

**EXEMPLE 18 : 8-ALLYL-2-STYRYL- 4H-1-BENZOPYRAN-4-ONE**

**STADE A : 3-ALLYL-2-HYDROXYBENZOATE DE METHYLE**

A l'abri de l'humidité, sous agitation et à 60°C, sont ajoutés, goutte à goutte, à une suspension d'hydrure de sodium (74 mmol) dans 60 ml de DMF anhydre,

10 g (66 mmol) de salicylate de méthyle en solution dans 10 ml de DMF anhydre. La température est maintenue 2 heures après la fin de l'addition ; le milieu réactionnel est ensuite additionné, goutte à goutte, de

6 ml (70 mmol) de bromure d'allyle et chauffé à nouveau pendant 2 heures. Après décomposition de l'excès d'hydrure de sodium par addition de méthanol, le milieu réactionnel est dilué par 50 ml d'éther éthylique puis versé sur 100 ml d'eau glacée. Après extraction de la phase aqueuse par l'éther éthylique, les phases organiques réunies sont lavées par une solution aqueuse de soude 1N, puis par une solution aqueuse saturée de NaCl et enfin séchées sur sulfate de sodium. L'élimination des solvants conduit à une huile orangée chauffée à 250°C pendant 30 minutes. La purification par distillation sous vide conduit à 11,44 g d'huile incolore.

Rdt : 90% Eb0,5 mmHg : 115-120°C
Analyses élémentaires : $C_{11}H_{12}O_3$, Mr=192,2

IR (KBr 1%, $cm^{-1}$) : 3300-3200, 2820, 1680.
RMN du $^1H$ ($CDCl_3$, δppm) : 3,45 (d, J = 6,5 Hz, 2H, $CH_2$-CH=$CH_2$);3,90 (s, 3H, $OCH_3$) ; 5,00-5,15 (dd, J = 16 Hz, 2H, CH=<u>$CH_2$</u>) ; 5,80-6,15 (m, 1H, $CH_2$-C<u>H</u>=$CH_2$) ; 6,75-7,75 (m, 3H, H4,5,6) ; 11,05 (s, 1H, OH) .

**STADE B: (3-ALLYL- 2 - HYDROXY BENZOYL) METHYLTRIPHENYLPHOSPHORANE**

Selon le protocole décrit au stade A de la préparation 3.
Cristaux orangés.

Rdt : 77% F : 176°C
Analyses élémentaires : $C_{29}H_{25}O_2P$, Mr=436,45
Calculé : C=79,81 %, H=5,77% ;
Trouvé : C=79,99%, H=5,96%.

IR et RMN du $^1H$ : Se reporter aux tableaux I et II.

**STADE C : 8-ALLYL- 2 - STYRYL - 4H - 1 - BENZOPYRAN - 4 - ONE**

Selon le protocole décrit au stade B de la préparation 3.
Cristaux beiges.

Rdt : 50% F : 142°C (Méthanol)
Analyses élémentaires : $C_{20}H_{16}O_2$, Mr=288,36
Calculé : C=83,31 %, H=5,89% ;
Trouvé : C=83,45%, H=5,75%.

IR et RMN du $^1H$ : Se reporter aux tableaux III et IV.

**EXEMPLE 19 : 8-ALLYL-3- METHYL -2- STYRYL -4H - 1 - BENZOPYRAN - 4 - ONE**

**STADE A : (1-(3-ALLYL-2-HYDROXY-BENZOYL)ETHYLIDENE)TRIPHENYL-PHOSPHORANE**

Selon le protocole décrit au stade B de l'exemple 18, en remplaçant l'iodure de méthyltriphénylphosphonium par l'iodure d'éthyltriphénylphosphonium.

Cristaux jaunes.

Rdt : 54% F : 152°C
Analyses élémentaires : $C_{30}H_{27}O_2P$, Mr=450,52
Calculé : C=79,98 %, H=6,04% ;
Trouvé : C=79,55%, H=6,06%.

IR et RMN du $^1H$ : Se reporter aux tableaux I et II.

**STADE B : 8-ALLYL- 3 - METHYL - 2 - STYRYL - 4H - 1 - BENZOPYRAN - 4 - ONE**

Selon le protocole décrit au stade B de la préparation 3.
Cristaux blancs.

Rdt : 60% F : 126°C (Méthanol)
Analyses élémentaires : $C_{21}H_{18}O_2$, Mr=302,37
Calculé : C=83,42 %, H=6,00% ;
Trouvé : C=82,79%, H=6,07%.

IR et RMN du $^1H$ : Se reporter aux tableaux III et IV.

**EXEMPLES 20 ET 21 :**

**6 - METHOXY - 8 - ALLYL - 2 - STYRYL - 4H - 1 - BENZOPYRAN - 4 - ONE (EXEMPLE 20)**

**8 - PROPYL - 2 - STYRYL - 4H - 1 - BENZOPYRAN - 4 - ONE (EXEMPLE 21)**

Ils sont obtenus en remplaçant au stade C de l'exemple 18 le (3-allyl-2-hydroxybenzoyl)-méthyltriphénylphosphorane par :
d'une part, (Exemple 20)
le (3-allyl-2-hydroxy-5-méthoxybenzoyl)-méthylènetriphénylphosphorane $C_{30}H_{27}O_3P$, Mr = 466,47,
dont les caractéristiques physiques sont les suivantes :
cristaux jaunes F : 186°C
IR et RMN du $^1H$ : Se reporter aux tableaux I et II.
d'autre part, (Exemple 21)
le (2-hydroxy-3-propyl)-méthylènetriphénylphosphorane $C_{29}H_{27}O_2P$, Mr = 438,45
obtenu comme au stade B de l'exemple 18 à partir du 2-hydroxy-3-propyl-benzoate de méthyle et dont les caractéristiques physiques sont les suivantes :
cristaux jaunes F : 142°C
IR et RMN du $^1H$ : Se reporter aux tableaux I et II.

**EXEMPLE 20 :**

cristaux blancs

Rdt : 70% F : 132°C (Méthanol)
Analyses élémentaires : $C_{21}H_{18}O_3$, Mr=318,37
Calculé : C=79,15 %, H=5,65% ;
Trouvé : C=79,40%, H=5,74%.

IR et RMN du $^1H$ : Se reporter aux tableaux III et IV.

**EXEMPLE 21 :**

cristaux blancs

Rdt : 70% F : 129°C (Méthanol)

Analyses élémentaires : $C_{20}H_{18}O_2$, Mr=290,36

Calculé : C=80,24 %, H=6,40% ;

Trouvé : C=79,86%, H=6,29%.

IR et RMN du $^1H$ : Se reporter aux tableaux III et IV.

TABLEAU I : CONSTANTES SPECTRALES IR($CM^{-1}$) DES (2-HYDROXYBENZOYL)-METHYLENE (ETHYLIDENE) TRIPHENYLPHOSPHORANES

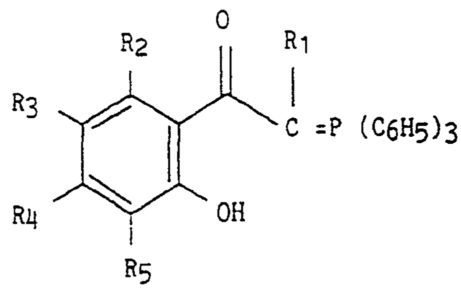

| N° | EXEMPLES | ν OH | ν C= O | ν C=C | γ C=H | ν C-P |
|---|---|---|---|---|---|---|
| 17 | $R_1=R_5=CH_3$, $R_2=R_3=R_4=H$ ; | 3200 | 1580 | 1520 | 760 et 700 | 690 |
| 18 | $R_1=R_2=R_3=R_4=H$, $R_5=CH_2-CH=CH_2$ ; | 3500 | 1595 | 1570 | 750 et 720 | 700 |
| 19 | $R_1=CH_3$, $R_5=CH_2-CH=CH_2$, $R_2=R_3=R_4=H$ ; | 3200 | 1600 | 1580 | 750 et 730 | 700 |
| 20 | $R_1=R_2=R_4=H$, $R_3=OCH_3$, $R_5=CH_2-CH=CH_2$ ; | 3300 | 1610 | 1590 | 850 | 700 |
| 21 | $R_1=R_2= R_3=R_4=H$, $R_5=CH_2\ CH_2CH_3$ ; | 3200 | 1610 | 1600 | 750 et 720 | 695 |

TABLEAU II

CONSTANTES SPECTRALES RMN DU $^{1}H$ DES (2-HYDROXYBENZOYL)-METHYLENE (ETHYLIDENE) TRIPHENYLPHOSPHORANES

(100 MHz, $CDCl_3$ , δ ppm)

| N° | EXEMPLES | $R_1$ | $R_2$ | $R_3$ | $R_4$ | $R_5$ |
|---|---|---|---|---|---|---|
| 19 | $R_1=R_5=CH_3$,<br>$R_2=R_3=R_4=H$ ; | 1,90<br>(d)<br>$^3J_{H-P}$ = 18 Hz | 7,30-7,80*<br>(m) | 2,20<br>(s) | 7,30-7,80*<br>(m) | 7,30-7,80*<br>(m) |
| 20 | $R_1=R_2=R_3=R_4=H$,<br>$R_5=CH_2-CH=CH_2$ | 7,00<br>(d)<br>$^2J_{H-P}$ = 32,4 Hz | 7,80-7,45*<br>(m) | 6,00 (1H, m)<br>5,05 (2H, d.d., J= 16 Hz)<br>3,40 (2H, d.e., J= 7 Hz) | 7,20<br>(d.e.)<br>$J_{4-5}$ = 7Hz | 6,80<br>(t)<br>$J_{5-6}$= 8 Hz |
| 21 | $R_1=CH_3,R_5=CH_2-CH=CH_2$,<br>$R_2=R_3=R_4=H$ ; | 1,85<br>(d)<br>$^3J_{H-P}$ = 17 Hz | 7,70-7,50*<br>(m) | 6,00 (1H, m)<br>5,10 (2H, d.d., J= 16 Hz)<br>3,35 (2H, d.e., J= 6 Hz) | 7,70-7,50*<br>(m) | 7,70-7,50*<br>(m) |

* Protons situés dans un massif complexe

TABLEAU III

CONSTANTES SPECTRALES IR($CM^{-1}$) DES 2-STYRYL-4H-1-BENZOPYRAN-4-ONES
($R_6 = R_7 = R_8 = H$)

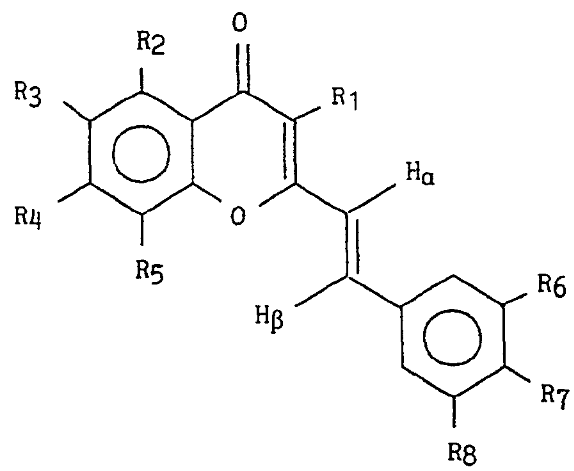

| N° | EXEMPLES | ν C = O | ν C=C |
|---|---|---|---|
| 17 | $R_1=R_5=CH_3$, $R_2=R_3=R_4=H$ ; | 1630 | 1610 et 1600 |
| 18 | $R_1=R_2=R_3=R_4=H$, $R_5=CH_2-CH=CH_2$ ; | 1630 | 1600 et 1590 |
| 19 | $R_1=CH_3$, $R_5=CH_2-CH=CH_2$, $R_2=R_3=R_4=H$ ; | 1620 | 1595 et 1580 |
| 20 | $R_1=R_2=R_4=H$, $R_3=OCH_3$, $R_5=CH_2-CH=CH_2$ ; | 1630 | 1600 et 1580 |
| 21 | $R_1=R_2= R_3=R_4=H$, $R_5=CH_2$ $CH_2CH_3$ ; | 1620 | 1610 et 1590 |

**TABLEAU IV**

**CONSTANTES SPECTRALES RMN DU 1 H DES 2-STYRYL-4H-1-BENZOPYRAN-4-ONES (100 MHz, $CDCl_3$ , δ ppm)**

| N° | EXEMPLES | $R_1$ | R2 | R3 R4 | R5 | Hα | Hβ H'ar |
|---|---|---|---|---|---|---|---|
| 17 | $R_1=R_5=CH_3$,<br>$R_2=R_3=R_4=H$ ; | 2,25<br>(s) | 8,15<br>(d.d.)<br>$J_{5-6}$ =7,5 Hz<br>$J_{5-7}$ =2,0 Hz | 7,70-7,15*<br>(m) | 2,60<br>(s) | 7,25<br>(d)<br>Jα-β=16 Hz | 7,70-7,15*<br>(m) |
| 18 | $R_1=R_2=R_3=R_4=H$,<br>$R_5=CH_2-CH=CH_2$ ; | 6,35<br>(s) | 8,10<br>(d.d.)<br>$J_{5-6}$ = 8 Hz<br>$J_{5-7}$ = 2 Hz | 7,70-7,25*<br>(m) | 3,75 (2H, d., J= 6,4 Hz)<br>5,20 (2H, d.d., J=16 Hz)<br>6,25-5,95 (1H, m) | 6,80<br>(d)<br>Jα-β=16 Hz | 7,40 7,70-7,25*<br>(d) (m)<br>Jα-β=16 Hz |
| 19 | $R_1=CH_3$, $R_5=CH_2$-<br>CH =$CH_2$,<br>R2=R3=R4=H ; | 2,25<br>(s) | 8,15<br>(d.d.)<br>$J_{5-6}$ = 8 Hz<br>$J_{5-7}$ = 2 Hz | 7,60-7,25*<br>(m) | 3,75 (2H, d., J= 6,2 Hz)<br>5,25 (2H, d.d., J=16 Hz)<br>6,30 (1H, m) | 7,30<br>(d)<br>Jα-β=16 Hz | 7,50 7,60-7,25*<br>(d) (m)<br>Jβ-α=16 Hz |
| 20 | $R_1=R_2=R_4=H$,<br>$R_3=OCH_3$, $R_5=CH_2$-<br>$CH=CH_2$ ; | 6,35<br>(s) | 7,65-7,15*<br>(m) | 3,90 7,65-<br>7,15*<br>(s) (m) | 3,70 (2H, d., J= 6,3 Hz)<br>5,30 (2H, d.d., J=16 Hz)<br>6,10-5,95 (1H, m) | 6,35<br>(d)<br>Jα-β=16 Hz | 7,40 7,65-7,15*<br>(d) (m)<br>Jβ-α=16 Hz |
| 21 | $R_1=R_2=R_3=R_4=H$,<br>$R_5=CH_2$ $CH_2CH_3$ ; | 6,35<br>(s) | 8,05<br>(d.d.)<br>$J_{5-6}$ = 8,0Hz<br>$J_{5-7}$ = 2 Hz | 8,05-7,25*<br>(m) | 2,95 (2H, t., J=7 Hz)<br>1,80 (2H, m, J=7Hz)<br>1,05 (2H, t, J=7Hz) | 6,80<br>(d)<br>Jα-β=16 Hz | 8,05-7,25*<br>(m) |

* Protons situés dans un massif complexe

## ETUDE PHARMACOLOGIQUE :

## EXEMPLE 22 : INHIBITION DE CROISSANCE DE LA LIGNEE L1210

La croissance de cette lignée leucémique de Souris est appréciée par la capacité des cellules à incorporer de la thymidine tritiée . Le taux d'incorporation est mesuré 24 heures après l'introduction du composé à tester dans le milieu de culture.
Le tableau V rassemble, pour chaque composé, la valeur de la concentration inhibant 50 % la croissance cellulaire.

TABLEAU V

| EXEMPLE N° | $IC_{50}$ (µM) 24 heures | EXEMPLE N° | $IC_{50}$ (µM) 24 heures |
|---|---|---|---|
| 1 | 12,50 | 17 | 7,50 |
| 2 | 6,40 | 18 | 5,85 |
| 3 | 5,00 | 19 | 6,60 |
| 4 | 12,00 | 20 | 3,00 |
| | | 21 | 7,25 |
| 7 | 8,50 | | |
| Référence : | | Acide Rétinoïque | 20,05 |

Cette étude montre que les exemples étudiés possèdent une activité supérieure à celle de l'acide rétinoïque.

## EXEMPLE 23 : ACTIVITE ANTITUMORALE SUR L'ADENOCARCINOME DU COLON C38 IN VIVO

Après implantation d'un fragment de tumeur (colon 38) en sous-cutané dans la région axillaire, est administrée, par voie intrapéritonéale, à un groupe d'animaux, les 2[e] et 4[e] jours après l'implantation, une solution du composé à tester. Au 20[e] jour, les animaux sont sacrifiés et le volume tumoral est déterminé.

Le rapport T/C % est calculé :

$$\frac{T}{C} = \frac{\text{Volume des tumeurs groupe expérimental}}{\text{Volume des tumeurs groupe contrôle}} \times 100$$

Le tableau VI rassemble les valeurs de T/C obtenues avec quelques composés.

TABLEAU VI

| EXEMPLE N° | Volume (mm3) tumoral après 20 jours | T/C% |
|---|---|---|
| 1 | 573 | 56 % |
| 2 | 356 | 35 % |
| Contrôle | 1027 | - |

**EXEMPLE 24 : DIFFERENTIATION CELLULAIRE**

Le test de différentiation cellulaire a été réalisé selon un protocole décrit par J.M.GALLUP et Coll (Proceedings of the Society for experimental biology and medecine, 186, 269-274, 1987).

Les cellules HL-60 (promyélocyte humain) à 2 x $10^5$ cellules/ml sont incubées pendant six jours avec les produits à tester. Elles sont ensuite comptées, centrifugées et remises en suspension à 2 x $10^6$ cellules/ml. Un aliquot de 0,5 ml de cellules est ensuite incubé avec 0,5 ml de NBT (Nitroblue Tetrazolium) à 0,2 % et 200 mg/ml de PMA (ester de phorbol) pendant 20 minutes à 37°C. Les cellules sont lavées avec du PBS puis elles sont numérées sur lames. Le tableau VII rassemble les résultats exprimés en pourcentage de cellules présentant des cristaux de NBT réduit (% de cellules NBT positives) à la concentration de 20 μM.

TABLEAU VII

| EXEMPLE N° | % de cellules NBT positives |
|---|---|
| 1 | 52 |
| 2 | 85 |
| 3 | 59 |
| 19 | 45 |

**EXEMPLE 25 : EXEMPLE DE COMPOSITION PHARMACEUTIQUE**

Comprimé dosé à 5 mg de
Formule de préparation pour 1000 comprimés :

| | |
|---|---|
| 6-méthoxy-8-allyl-2-styryl-4H-1-benzopyran-4-one | 5 g |
| Amidon de blé | 20 g |
| Amidon de maïs | 20 g |
| Lactose | 75 g |
| Stéarate de magnésium | 2 g |
| Silice | 1 g |
| Hydroxypropyl cellulose | 2 g |

**Revendications**

1. Composés de formule générale (I) :

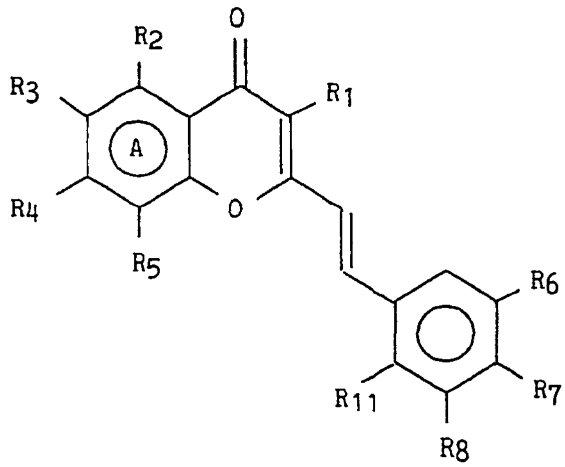

(I)

dans laquelle :

$R_1$ représente un atome d'hydrogène, un groupement alcoyle inférieur, hydroxyle ou alcoyloxyle inférieur,

$R_6$, $R_7$, $R_8$, $R_{11}$ identiques ou différents représentent indépendamment l'un de l'autre un atome d'halogène, un groupement hydroxyle, alcoyloxyle inférieur, thio, alcoylthio inférieur, sulfonyle, alcoylsulfonyle inférieur, ou un groupement de formule (G) :

D-(A)m-(O-CO)p-(B)n **(G)**

avec n, m, p, identiques ou différents, égaux à 0 ou 1, étant entendu que si p = 0, alors m = 0 ;

ou bien $R_8$ et $R_{11}$ forment avec le cycle aromatique qui les porte un groupement naphtyl,

A et B identiques ou différents représentent un groupe alcoyle inférieur linéaire ou ramifié ou alcényle inférieur linéaire ou ramifié ;

D représente un atome d'hydrogène ou un groupement de formule :

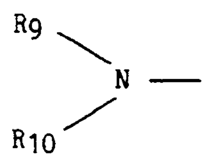

avec $R_9$ et $R_{10}$ identiques ou différents, représentant indépendamment l'un de l'autre, un atome d'hydrogène, un groupement alcoyle inférieur, un groupement acyl alcoyl inférieur ou formant avec l'atome d'azote qui les porte un système hétérocyclique mono ou bicyclique comprenant de 4 à 10 sommets parmi lesquels on peut trouver un ou deux hétéroatomes choisis parmi oxygène, soufre ou azote ;

$R_2$, $R_3$, $R_4$, $R_5$ identiques ou différents représentent un atome d'halogène, ou un groupement hydroxyle, alcoyloxy inférieur ou un groupement de formule (G) tel que ci-dessus défini,

– étant entendu que :

. l'un au moins de $R_3$ ou $R_5$ représente un groupement de formule (G) différent de l'hydrogène,

. si $R_3$ représente un groupement de formule (G) avec n = 0 et p = 1, alors l'un au moins de $R_2$, $R_4$, $R_5$ est différent de l'hydrogène,

. si $R_5$ représente un groupement méthyle ou si $R_3$ représente un groupement alcoyle inférieur, alors l'un au moins des trois autres substituants du cycle aromatique A est différent de l'hydrogène,

ou bien $R_3$ et $R_4$ forment ensemble avec le cycle aromatique qui les porte un système bi ou tricyclique de 9 à 15 atomes, saturé ou non susceptible d'inclure dans un squelette carboné un ou plusieurs hétéroatomes choisis parmi l'oxygène, le soufre ou l'azote étant entendu que :

– lorsque $R_3$ et $R_4$ forment avec le cycle aromatique qui les porte un système benzofurannique ou benzopyrannique saturés ou non, $R_2$ et $R_5$ ne peuvent représenter un groupement hydroxyle ou alkoxy inférieur lorsque $R_6$, $R_7$, $R_8$ et $R_{11}$ simultanément représentent un atome d'hydrogène,

– et lorsque $R_3$ et $R_4$ forment avec le cycle aromatique qui les porte un système benzofurannique

non saturé, $R_7$ ne peut représenter un groupement alkyle lorsque $R_6$, $R_8$ et $R_{11}$ représentent simultanément un atome d'hydrogène, $R_2$ un groupement alkoxy et $R_5$ un groupement alkoxy ou un atome d'hydrogène,

leurs isomères, énantiomères, diastéréoisomères ainsi que, lorsque le dérivé de formule (I) comprend un groupement acide, ses sels d'addition à une base pharmaceutiquement acceptable et lorsqu'il comprend un groupement basique, ses sels d'addition à un acide pharmaceutiquement acceptable, étant entendu que par groupements alcoyle inférieur, alcényle inférieur et alcoyloxy inférieur on entend des groupements linéaires ou ramifiés comprenant de 1 à 6 atomes de carbone.

**2.** Composés de formule (I) selon la revendication 1 pour lesquels la configuration de la double liaison du groupement styryle est (E).

**3.** Composés de formule (I) selon l'une des revendications 1 ou 2 pour lesquels $R_3$ représente un groupement de formule (G) dans lequel p = 1 leurs isomères et lorsque le dérivé de formule (I) comprend un groupement acide, ses sels d'addition à une base pharmaceutiquement acceptable et lorsqu'il contient un groupement basique, ses sels d'addition à un acide pharmaceutiquement acceptable.

**4.** Composés de formule (I) selon l'une des revendications 1, 2 ou 3 pour lesquels $R_3$ représente un groupement méthoxy carbonyle, leurs isomères, et lorsque le dérivé de formule (I) comprend un groupement acide, ses sels d'addition à une base pharmaceutiquement acceptable et lorsqu'il contient un groupement basique, ses sels d'addition à un acide pharmaceutiquement acceptable.

**5.** Composés de formule (I) selon l'une des revendications 1 ou 2 pour lesquels $R_5$ représente un groupement de formule (G) dans lequel n=p=1, leurs isomères et lorsque le dérivé de formule (I) comprend un groupement acide, ses sels d'addition à une base pharmaceutiquement acceptable et lorsqu'il contient un groupement basique, ses sels d'addition à un acide pharmaceutiquement acceptable.

**6.** Composés de formule (I) selon l'une des revendications 1, 2 ou 4 pour lesquels $R_5$ représente un groupement carboxyméthyle, leurs isomères, et lorsque le dérivé de formule (I) comprend un groupement acide, leurs sels d'addition à une base pharmaceutiquement acceptable et lorsque la molécule contient un groupement basique, leurs sels d'addition à un acide pharmaceutiquement acceptable.

**7.** Composés de formule (I) selon l'une des revendications 1 ou 2 pour lesquels $R_3$ et $R_4$ forment avec le cycle aromatique qui les porte un système benzofurannique, leurs isomères, et lorsque le dérivé de formule (I) comprend un groupement acide, ses sels d'addition à une base pharmaceutiquement acceptable et lorsqu'il contient un groupement basique, ses sels d'addition à un acide pharmaceutiquement acceptable.

**8.** Composé de formule (I) selon l'une des revendications 1 ou 2 qui est le 5,7-diméthoxy-4-oxo-2-(3,4-diméthoxy styryl)-4H-1-benzopyrane-6-carboxylate de méthyle, ses isomères.

**9.** Composé de formule (I) selon l'une des revendications 1 ou 2 qui est le 6-méthoxy 8-allyl-2-styryl-4H-1 benzopyrane 4-one, ses isomères.

**10.** Composé de formule (I) selon l'une des revendications 1 ou 2 qui est la 4-méthoxy-7-(3,4-diméthoxystyryl)-5H-furo(3,2-g)benzopyran-5-one et ses isomères.

**11.** Procédé de préparation des dérivés de formule (I) caractérisés :

– ou bien par le fait que l'on condense par chauffage à reflux du solvant un dérivé de formule (II),

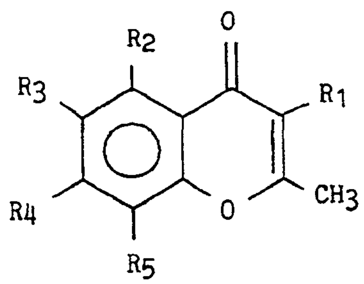

(II)

dans lequel $R_1$, $R_2$, $R_3$, $R_4$ et $R_5$ possèdent la même signification que dans la formule **(I)**, avec un aldéhyde benzoïque substitué (III) dans lequel $R_6$, $R_7$, $R_8$ et $R_{11}$ possèdent la même signification que dans le formule (I),

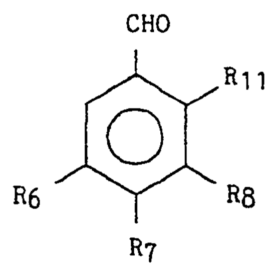

(III)

en présence d'un agent basique dans un solvant polaire, pour conduire après refroidissement et filtration du milieu réactionnel à un composé de formule (I) ;
– ou bien par le fait que l'on traite deux molécules d'halogénure de triphénylalcoylphosphonium (ou triphénylalcoyloxy methylphosphonium) de formule (IV) :

$$R_1 - CH_2 - P^{\oplus}(C_6H_5)_3 X^{\ominus} \qquad \textbf{(IV)}$$

dans laquelle $R_1$ représente un atome d'hydrogène, un alcoyle inférieur, un alcoyloxyle inférieur et $X^{\ominus}$ l'anion d'un hydracide, en suspension dans un solvant organique, par une base forte la solution obtenue après un contact de plusieurs heures étant, à son tour, traitée sous atmosphère inerte par une molécule de 2-hydroxy benzoate d'alcoyle de formule (V) :

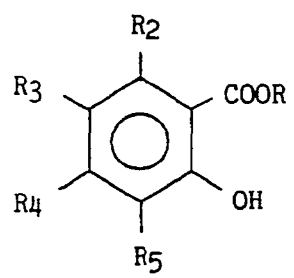

(V)

dans lequel $R_2$, $R_3$, $R_4$ et $R_5$ possèdent la même signification que dans la formule (I) et R représente un alcoyle inférieur,
en solution dans le solvant précédemment choisi, le milieu obtenu étant ensuite chauffé pour obtenir après filtration du milieu réactionnel et élimination sous vide des solvants un dérivé de formule (VI) :

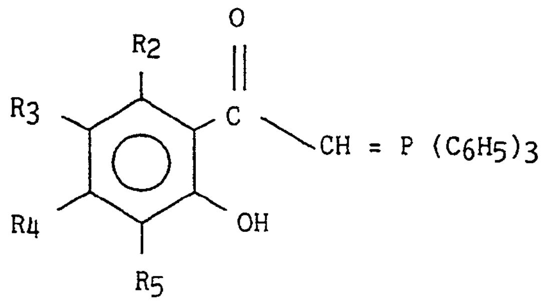

(VI)

dans lequel $R_2$, $R_3$, $R_4$ et $R_5$ possèdent la même signification que précédemment, qui, placé dans la pyridine anhydre et sous atmosphère inerte, est additionné du chlorure de l'acide cinnamique de formule (VII) :

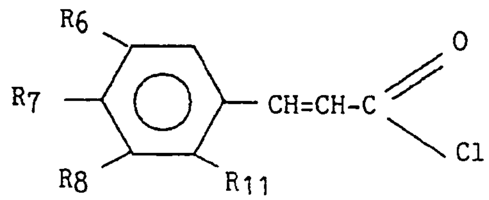

(VII)

dans lequel $R_6$, $R_7$, $R_8$ et $R_{11}$ possèdent la même signification que dans la formule (I), et, chauffé à une température préférentielle de 60°C pendant une journée environ pour conduire, après élimination du solvant, reprise par un solvant organique approprié et extractions successives en milieu alcalin et enfin élimination des solvants, à un composé de formule (I) qui quel que soit le procédé selon lequel il a été obtenu, est, si on le désire, séparé en ses stéréoisomères, diastéréoisomères, énantiomères par une technique de cristallisation ou de chromatographie, puis, si on le désire, lorsque le dérivé de formule (I) comprend un groupement acide ou basique, salifié respectivement par une base ou un acide pharmaceutiquement acceptables.

**12.** Procédé de préparation selon la revendication 11 dans lequel à une suspension de deux molécules d'un dérivé de formule (IV) selon la revendication 11 dans du tétrahydrofuranne on additionne une solution de n-butyllithium dans l'hexane, pour conduire, après plusieurs heures de contact à température ambiante à une solution qui est traitée sous atmosphère inerte par une molécule de dérivé (V) selon la revendication 11 en solution dans le tétrahydrofuranne le milieu obtenu étant ensuite chauffé à une température comprise entre 50 et 60°C pour obtenir après filtration du milieu réactionnel et élimination sous vide des solvants un dérivé de formule (VI) selon la revendication 11 qui, placé dans la pyridine anhydre et sous atmosphère inerte est additionné d'un dérivé de formule (VII) selon la revendication 11, pour conduire après chauffage à une température de 60 °C pendant une journée, élimination du solvant reprise par un solvant organique approprié et extractions successives en milieu alcalin et enfin élimination des solvants, à un composé de formule (I)
qui quel que soit le procédé selon lequel il a été obtenu, est, si on le désire, séparé en ses stéréoisomères, diastéréoisomères, énantiomères par une technique de cristallisation ou de chromatographie, puis, si on le désire, lorsque le dérivé de formule (I) comprend un groupement acide ou basique, salifié respectivement par une base ou un acide pharmaceutiquement acceptables.

**13.** Composition pharmaceutique contenant comme principe actif au moins un composé selon l'une des revendications 1 à 10 en combinaison avec un ou plusieurs excipients ou véhcules inertes, non toxiques, pharmaceutiquement acceptables.

**14.** Composition pharmaceutique selon la revendication 13 contenant comme principe actif au moins un composé selon l'une des revendications 1 à 10 utilisables dans le traitement des cancers.

**Revendications pour l'Etat contractant suivant: ES**

**1.** Procédé de préparation des composés de formule générale (I) :

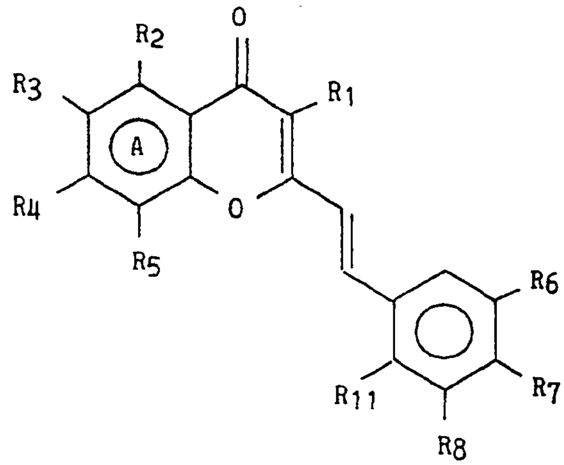

(I)

dans laquelle :

$R_1$ représente un atome d'hydrogène, un groupement alcoyle inférieur, hydroxyle ou alcoyloxyle inférieur,

$R_6$, $R_7$, $R_8$, $R_{11}$ identiques ou différents représentent indépendamment l'un de l'autre un atome d'halogène, un groupement hydroxyle, alcoyloxyle inférieur, thio, alcoylthio inférieur, sulfonyle, alcoylsulfonyle inférieur, ou un groupement de formule (G) :

D-(A)m-(O-CO)p-(B)n **(G)**

avec n, m, p, identiques ou différents, égaux à 0 ou 1, étant entendu que si p = 0, alors m = 0 ;

ou bien $R_8$ et $R_{11}$ forment avec le cycle aromatique qui les porte un groupement naphtyl,

A et B identiques ou différents représentent un groupe alcoyle inférieur linéaire ou ramifié ou alcényle inférieur linéaire ou ramifié ;

D représente un atome d'hydrogène ou un groupement de formule :

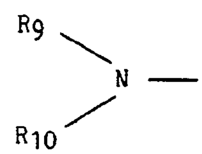

avec $R_9$ et $R_{10}$ identiques ou différents, représentant indépendamment l'un de l'autre, un atome d'hydrogène, un groupement alcoyle inférieur, un groupement acyl alcoyl inférieur ou formant avec l'atome d'azote qui les porte un système hétérocyclique mono ou bicyclique comprenant de 4 à 10 sommets parmi lesquels on peut trouver un ou deux hétéroatomes choisis parmi oxygène, soufre ou azote ;

$R_2$, $R_3$, $R_4$, $R_5$ identiques ou différents représentent un atome d'halogène, ou un groupement hydroxyle, alcoyloxy inférieur ou un groupement de formule (G) tel que ci-dessus défini,

– étant entendu que :

. l'un au moins de $R_3$ ou $R_5$ représente un groupement de formule (G) différent de l'hydrogène,

. si $R_3$ représente un groupement de formule (G) avec n = 0 et p = 1, alors l'un au moins de $R_2$, $R_4$, $R_5$ est différent de l'hydrogène,

. si $R_5$ représente un groupement méthyle ou si $R_3$ représente un groupement alcoyle inférieur, alors l'un au moins des trois autres substituants du cycle aromatique A est différent de l'hydrogène,

ou bien $R_3$ et $R_4$ forment ensemble avec le cycle aromatique qui les porte un système bi ou tricyclique de 9 à 15 atomes, saturé ou non susceptible d'inclure dans un squelette carboné un ou plusieurs hétéroatomes choisis parmi l'oxygène, le soufre ou l'azote étant entendu que :

– lorsque $R_3$ et $R_4$ forment avec le cycle aromatique qui les porte un système benzofurannique ou benzopyrannique saturés ou non, $R_2$ et $R_5$ ne peuvent représenter un groupement hydroxyle ou alkoxy inférieur lorsque $R_6$, $R_7$, $R_8$ et $R_{11}$ simultanément représentent un atome d'hydrogène,

– et lorsque $R_3$ et $R_4$ forment avec le cycle aromatique qui les porte un système benzofurannique non saturé, $R_7$ ne peut représenter un groupement alkyle lorsque $R_6$, $R_8$ et $R_{11}$ représentent simultanément un atome d'hydrogène, $R_2$ un groupement alkoxy et $R_5$ un groupement alkoxy ou un atome d'hydrogène,

leurs isomères, énantiomères, diastéréoisomères ainsi que, lorsque le dérivé de formule (I) comprend un

groupement acide, ses sels d'addition à une base pharmaceutiquement acceptable et lorsqu'il comprend un groupement basique, ses sels d'addition à un acide pharmaceutiquement acceptable, étant entendu que par groupements alcoyle inférieur, alcényle inférieur et alcoyloxy inférieur on entend des groupements linéaires ou ramifiés comprenant de 1 à 6 atomes de carbone,
caractérisé :

– ou bien par le fait que l'on condense par chauffage à reflux du solvant un dérivé de formule (II),

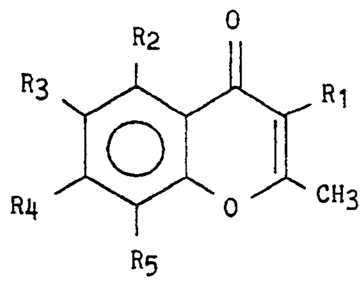

(II)

dans lequel $R_1$, $R_2$, $R_3$, $R_4$ et $R_5$ possèdent la même signification que dans la formule (I) ,
avec un aldéhyde benzoïque substitué (III) dans lequel $R_6$, $R_7$, $R_8$ et $R_{11}$ possèdent la même signification que dans le formule (I),

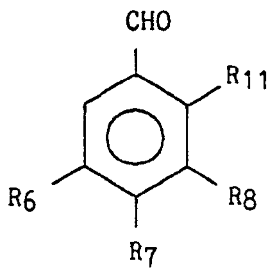

(III)

en présence d'un agent basique dans un solvant polaire, pour conduire après refroidissement et filtration du milieu réactionnel à un composé de formule (I) ;

– ou bien par le fait que l'on traite deux molécules d'halogénure de triphénylalcoylphosphonium (ou triphénylalcoyloxy methylphosphonium) de formule (IV) :

$$R_1 - CH_2 - P^{\oplus} (C_6H_5)_3 X^{\ominus} \qquad \textbf{(IV)}$$

dans laquelle $R_1$ représente un atome d'hydrogène , un alcoyle inférieur, un alcoyloxyle inférieur et $X^{\ominus}$ l'anion d'un hydracide, en suspension dans un solvant organique, par une base forte la solution obtenue après un contact de plusieurs heures étant, à son tour, traitée sous atmosphère inerte par une molécule de 2-hydroxy benzoate d'alcoyle de formule (V) :

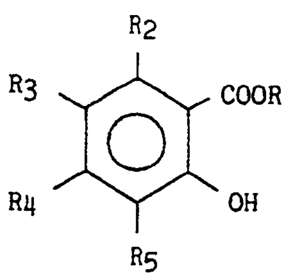

(V)

dans lequel $R_2$, $R_3$, $R_4$ et $R_5$ possèdent la même signification que dans la formule (I) et R représente un alcoyle inférieur,
en solution dans le solvant précédemment choisi, le milieu obtenu étant ensuite chauffé pour obtenir après filtration du milieu réactionnel et élimination sous vide des solvants un dérivé de formule (VI) :

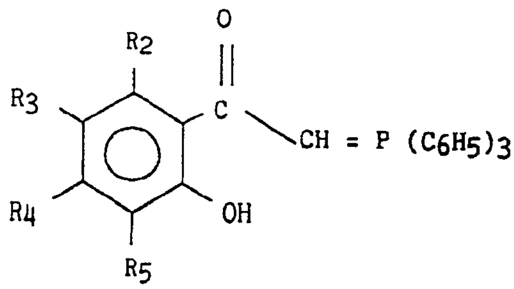

(VI)

dans lequel $R_2$, $R_3$, $R_4$ et $R_5$ possèdent la même signification que précédemment,
qui, placé dans la pyridine anhydre et sous atmosphère inerte, est additionné du chlorure de l'acide cinnamique de formule (VII) :

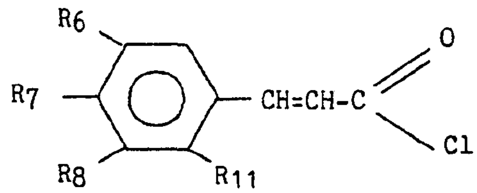

(VII)

dans lequel $R_6$, $R_7$, $R_8$ et $R_{11}$ possèdent la même signification que dans la formule (I),
et, chauffé à une température préférentielle de 60°C pendant une journée environ pour conduire, après élimination du solvant, reprise par un solvant organique approprié et extractions successives en milieu alcalin et enfin élimination des solvants, à un composé de formule (I)
qui quel que soit le procédé selon lequel il a été obtenu, est, si on le désire, séparé en ses stéréoisomères, diastéréoisomères, énantiomères par une technique de cristallisation ou de chromatographie, puis, si on le désire, lorsque le dérivé de formule (I) comprend un groupement acide ou basique, salifié respectivement par une base ou un acide pharmaceutiquement acceptables.

2. Procédé de préparation selon la revendication 1 dans lequel à une suspension de deux molécules d'un dérivé de formule (IV) selon la revendication 1 dans du tétrahydrofuranne on additionne une solution de n-butyllithium dans l'hexane, pour conduire, après plusieurs heures de contact à température ambiante à une solution qui est traitée sous atmosphère inerte par une molécule de dérivé (V) selon la revendication 1 en solution dans le tétrahydrofuranne le milieu obtenu étant ensuite chauffé à une température comprise entre 50 et 60°C pour obtenir après filtration du milieu réactionnel et élimination sous vide des solvants un dérivé de formule (VI) selon la revendication 1 qui, placé dans la pyridine anhydre et sous atmosphère inerte est additionné d'un dérivé de formule (VII) selon la revendication 1, pour conduire après chauffage à une température de 60 °C pendant une journée, élimination du solvant reprise par un solvant organique approprié et extractions successives en milieu alcalin et enfin élimination des solvants, à un composé de formule (I)
qui quel que soit le procédé selon lequel il a été obtenu, est, si on le désire, séparé en ses stéréoisomères, diastéréoisomères, énantiomères par une technique de cristallisation ou de chromatographie, puis, si on le désire, lorsque le dérivé de formule (I) comprend un groupement acide ou basique, salifié respectivement par une base ou un acide pharmaceutiquement acceptables.

3. Procédé de préparation de composés de formule (I) selon l'une des revendications 1 ou 2 pour lesquels la configuration de la double liaison du groupement styryle est (E).

4. Procédé de préparation de composés de formule (I) selon l'une des revendications 1 ou 2 pour lesquels $R_3$ représente un groupement de formule (G) dans lequel p = 1 leurs isomères et lorsque le dérivé de formule (I) comprend un groupement acide, ses sels d'addition à une base pharmaceutiquement acceptable et lorsqu'il contient un groupement basique, ses sels d'addition à un acide pharmaceutiquement acceptable.

5. Procédé de préparation de composés de formule (I) selon l'une des revendications 1, 2 pour lesquels $R_3$

représente un groupement méthoxy carbonyle, leurs isomères, et lorsque le dérivé de formule (I) comprend un groupement acide, ses sels d'addition à une base pharmaceutiquement acceptable et lorsqu'il contient un groupement basique, ses sels d'addition à un acide pharmaceutiquement acceptable.

6. Procédé de préparation de composés de formule (I) selon l'une des revendications 1 ou 2 pour lesquels $R_5$ représente un groupement de formule (G) dans lequel n=p=1, leurs isomères et lorsque le dérivé de formule (I) comprend un groupement acide, ses sels d'addition à une base pharmaceutiquement acceptable et lorsqu'il contient un groupement basique, ses sels d'addition à un acide pharmaceutiquement acceptable.

7. Procédé de préparation de composés de formule (I) selon l'une des revendications 1, 2 pour lesquels $R_5$ représente un groupement carboxyméthyle, leurs isomères, et lorsque le dérivé de formule (I) comprend un groupement acide, leurs sels d'addition à une base pharmaceutiquement acceptable et lorsque la molécule contient un groupement basique, leurs sels d'addition à un acide pharmaceutiquement acceptable.

8. Procédé de préparation de composés de formule (I) selon l'une des revendications 1 ou 2 pour lesquels $R_3$ et $R_4$ forment avec le cycle aromatique qui les porte un système benzofurannique, leurs isomères, et lorsque le dérivé de formule (I) comprend un groupement acide, ses sels d'addition à une base pharmaceutiquement acceptable et lorsqu'il contient un groupement basique, ses sels d'addition à un acide pharmaceutiquement acceptable.

9. Procédé de préparation de composé de formule (I) selon l'une des revendications 1 ou 2 qui est le 5,7-diméthoxy-4-oxo-2-(3,4-diméthoxy styryl)-4H-1-benzopyrane-6-carboxylate de méthyle, ses isomères.

10. Procédé de préparation de composé de formule (I) selon l'une des revendications 1 ou 2 qui est le 6-méthoxy 8-allyl-2-styryl-4H-1 benzopyrane 4-one, ses isomères.

11. Procédé de préparation de composé de formule (I) selon l'une des revendications 1 ou 2 qui est la 4-méthoxy-7-(3,4-diméthoxystyryl)-5H-furo(3,2-g)benzopyran-5-one et ses isomères.

**Revendications pour l'Etat contractant suivant: GR**

1. Procédé de préparation des composés de formule générale (I) :

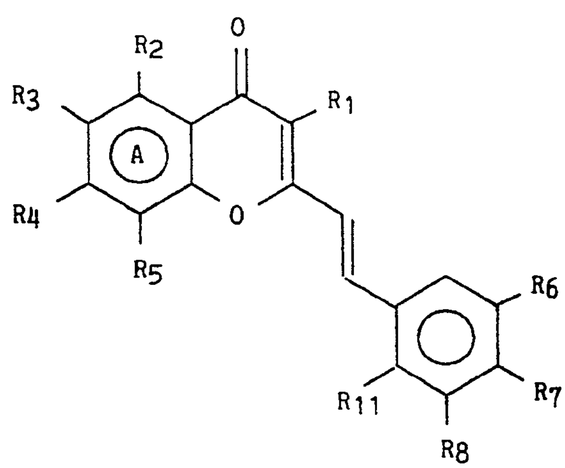

(I)

dans laquelle :

$R_1$ représente un atome d'hydrogène, un groupement alcoyle inférieur, hydroxyle ou alcoyloxyle inférieur,

$R_6$, $R_7$, $R_8$, $R_{11}$ identiques ou différents représentent indépendamment l'un de l'autre un atome d'halogène, un groupement hydroxyle, alcoyloxyle inférieur, thio, alcoylthio inférieur, sulfonyle, alcoylsulfonyle inférieur, ou un groupement de formule (G) :

D-(A)m-(O-CO)p-(B)n **(G)**

avec n, m, p, identiques ou différents, égaux à 0 ou 1, étant entendu que si p = 0, alors m = 0 ;

ou bien $R_8$ et $R_{11}$ forment avec le cycle aromatique qui les porte un groupement naphtyl,
A et B identiques ou différents représentent un groupe alcoyle inférieur linéaire ou ramifié ou alcényle inférieur linéaire ou ramifié ;
D représente un atome d'hydrogène ou un groupement de formule :

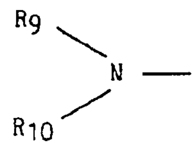

avec $R_9$ et $R_{10}$ identiques ou différents, représentant indépendamment l'un de l'autre, un atome d'hydrogène, un groupement alcoyle inférieur, un groupement acyl alcoyl inférieur ou formant avec l'atome d'azote qui les porte un système hétérocyclique mono ou bicyclique comprenant de 4 à 10 sommets parmi lesquels on peut trouver un ou deux hétéroatomes choisis parmi oxygène, soufre ou azote ;
$R_2$, $R_3$, $R_4$, $R_5$ identiques ou différents représentent un atome d'halogène, ou un groupement hydroxyle, alcoyloxy inférieur ou un groupement de formule (G) tel que ci-dessus défini,

- étant entendu que :
  . l'un au moins de $R_3$ ou $R_5$ représente un groupement de formule (G) différent de l'hydrogène,
  . si $R_3$ représente un groupement de formule (G) avec n = 0 et p = 1, alors l'un au moins de $R_2$, $R_4$, $R_5$ est différent de l'hydrogène,
  . si $R_5$ représente un groupement méthyle ou si $R_3$ représente un groupement alcoyle inférieur, alors l'un au moins des trois autres substituants du cycle aromatique A est différent de l'hydrogène,
  ou bien $R_3$ et $R_4$ forment ensemble avec le cycle aromatique qui les porte un système bi ou tricyclique de 9 à 15 atomes, saturé ou non susceptible d'inclure dans un squelette carboné un ou plusieurs hétéroatomes choisis parmi l'oxygène, le soufre ou l'azote étant entendu que :
  - lorsque $R_3$ et $R_4$ forment avec le cycle aromatique qui les porte un système benzofurannique ou benzopyrannique saturés ou non, $R_2$ et $R_5$ ne peuvent représenter un groupement hydroxyle ou alkoxy inférieur lorsque $R_6$, $R_7$, $R_8$ et $R_{11}$ simultanément représentent un atome d'hydrogène,
  - et lorsque $R_3$ et $R_4$ forment avec le cycle aromatique qui les porte un système benzofurannique non saturé, $R_7$ ne peut représenter un groupement alkyle lorsque $R_6$, $R_8$ et $R_{11}$ représentent simultanément un atome d'hydrogène, $R_2$ un groupement alkoxy et $R_5$ un groupement alkoxy ou un atome d'hydrogène,

leurs isomères, énantiomères, diastéréoisomères ainsi que, lorsque le dérivé de formule (I) comprend un groupement acide, ses sels d'addition à une base pharmaceutiquement acceptable et lorsqu'il comprend un groupement basique, ses sels d'addition à un acide pharmaceutiquement acceptable, étant entendu que par groupements alcoyle inférieur, alcényle inférieur et alcoyloxy inférieur on entend des groupements linéaires ou ramifiés comprenant de 1 à 6 atomes de carbone,
caractérisé :

- ou bien par le fait que l'on condense par chauffage à reflux du solvant un dérivé de formule (II),

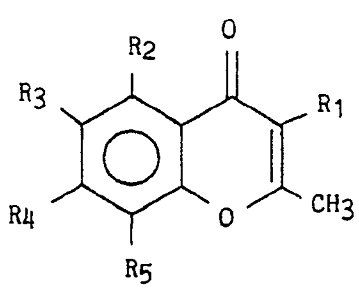

(II)

dans lequel $R_1$, $R_2$, $R_3$, $R_4$ et $R_5$ possèdent la même signification que dans la formule (I) ,
avec un aldéhyde benzoïque substitué (III) dans lequel $R_6$, $R_7$, $R_8$ et $R_{11}$ possèdent la même signification que dans le formule (I),

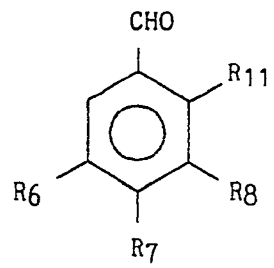

(III)

en présence d'un agent basique dans un solvant polaire, pour conduire après refroidissement et filtration du milieu réactionnel à un composé de formule (I) ;

– ou bien par le fait que l'on traite deux molécules d'halogénure de triphénylalcoylphosphonium (ou triphénylalcoyloxy methylphosphonium) de formule (IV) :

$$R_1 - CH_2 - P^{\oplus} (C_6H_5)_3 X^{\ominus} \qquad \textbf{(IV)}$$

dans laquelle $R_1$ représente un atome d'hydrogène , un alcoyle inférieur, un alcoyloxyle inférieur et $X^{\ominus}$ l'anion d'un hydracide, en suspension dans un solvant organique, par une base forte la solution obtenue après un contact de plusieurs heures étant, à son tour, traitée sous atmosphère inerte par une molécule de 2-hydroxy benzoate d'alcoyle de formule (V) :

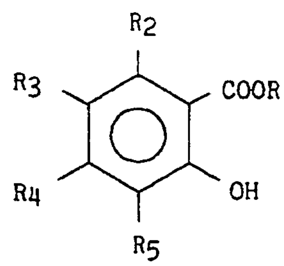

(V)

dans lequel $R_2$, $R_3$, $R_4$ et $R_5$ possèdent la même signification que dans la formule (I) et R représente un alcoyle inférieur,

en solution dans le solvant précédemment choisi, le milieu obtenu étant ensuite chauffé pour obtenir après filtration du milieu réactionnel et élimination sous vide des solvants un dérivé de formule (VI) :

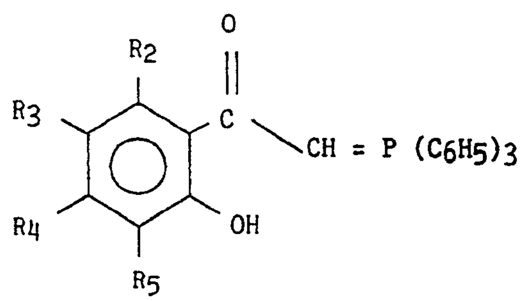

(VI)

dans lequel $R_2$, $R_3$, $R_4$ et $R_5$ possèdent la même signification que précédemment,

qui, placé dans la pyridine anhydre et sous atmosphère inerte, est additionné du chlorure de l'acide cinnamique de formule (VII) :

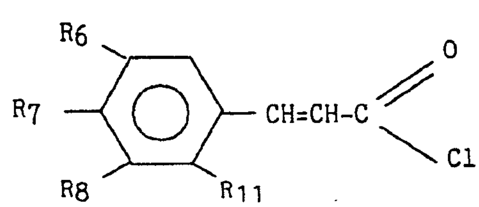

(VII)

dans lequel $R_6$, $R_7$, $R_8$ et $R_{11}$ possèdent la même signification que dans la formule (I), et, chauffé à une température préférentielle de 60°C pendant une journée environ pour conduire, après élimination du solvant, reprise par un solvant organique approprié et extractions successives en milieu alcalin et enfin élimination des solvants, à un composé de formule (I) qui quel que soit le procédé selon lequel il a été obtenu, est, si on le désire, séparé en ses stéréoisomères, diastéréoisomères, énantiomères par une technique de cristallisation ou de chromatographie, puis, si on le désire, lorsque le dérivé de formule (I) comprend un groupement acide ou basique, salifié respectivement par une base ou un acide pharmaceutiquement acceptables.

2. Procédé de préparation selon la revendication 1 dans lequel à une suspension de deux molécules d'un dérivé de formule (IV) selon la revendication 1 dans du tétrahydrofuranne on additionne une solution de n-butyllithium dans l'hexane, pour conduire, après plusieurs heures de contact à température ambiante à une solution qui est traitée sous atmosphère inerte par une molécule de dérivé (V) selon la revendication 1 en solution dans le tétrahydrofuranne le milieu obtenu étant ensuite chauffé à une température comprise entre 50 et 60°C pour obtenir après filtration du milieu réactionnel et élimination sous vide des solvants un dérivé de formule (VI) selon la revendication 1 qui, placé dans la pyridine anhydre et sous atmosphère inerte est additionné d'un dérivé de formule (VII) selon la revendication 1, pour conduire après chauffage à une température de 60 °C pendant une journée, élimination du solvant reprise par un solvant organique approprié et extractions successives en milieu alcalin et enfin élimination des solvants, à un composé de formule (I)
qui quel que soit le procédé selon lequel il a été obtenu, est, si on le désire, séparé en ses stéréoisomères, diastéréoisomères, énantiomères par une technique de cristallisation ou de chromatographie, puis, si on le désire, lorsque le dérivé de formule (I) comprend un groupement acide ou basique, salifié respectivement par une base ou un acide pharmaceutiquement acceptables.

3. Procédé de préparation de composés de formule (I) selon l'une des revendications 1 ou 2 pour lesquels la configuration de la double liaison du groupement styryle est (E).

4. Procédé de préparation de composés de formule (I) selon l'une des revendications 1 ou 2 pour lesquels $R_3$ représente un groupement de formule (G) dans lequel p = 1 leurs isomères et lorsque le dérivé de formule (I) comprend un groupement acide, ses sels d'addition à une base pharmaceutiquement acceptable et lorsqu'il contient un groupement basique, ses sels d'addition à un acide pharmaceutiquement acceptable.

5. Procédé de préparation de composés de formule (I) selon l'une des revendications 1, 2 pour lesquels $R_3$ représente un groupement méthoxy carbonyle, leurs isomères, et lorsque le dérivé de formule (I) comprend un groupement acide, ses sels d'addition à une base pharmaceutiquement acceptable et lorsqu'il contient un groupement basique, ses sels d'addition à un acide pharmaceutiquement acceptable.

6. Procédé de préparation de composés de formule (I) selon l'une des revendications 1 ou 2 pour lesquels $R_5$ représente un groupement de formule (G) dans lequel n=p=1, leurs isomères et lorsque le dérivé de formule (I) comprend un groupement acide, ses sels d'addition à une base pharmaceutiquement acceptable et lorsqu'il contient un groupement basique, ses sels d'addition à un acide pharmaceutiquement acceptable.

7. Procédé de préparation de composés de formule (I) selon l'une des revendications 1, 2 pour lesquels $R_5$ représente un groupement carboxyméthyle, leurs isomères, et lorsque le dérivé de formule (I) comprend un groupement acide, leurs sels d'addition à une base pharmaceutiquement acceptable et lorsque la molécule contient un groupement basique, leurs sels d'addition à un acide pharmaceutiquement acceptable.

8. Procédé de préparation de composés de formule (I) selon l'une des revendications 1 ou 2 pour lesquels $R_3$ et $R_4$ forment avec le cycle aromatique qui les porte un système benzofurannique, leurs isomères, et lorsque le dérivé de formule (I) comprend un groupement acide, ses sels d'addition à une base pharmaceutiquement acceptable et lorsqu'il contient un groupement basique, ses sels d'addition à un acide pharmaceutiquement acceptable.

9. Procédé de préparation de composé de formule (I) selon l'une des revendications 1 ou 2 qui est le 5,7-diméthoxy-4-oxo-2-(3,4-diméthoxy styryl)-4H-1-benzopyrane-6-carboxylate de méthyle, ses isomères.

**10.** Procédé de préparation de composé de formule (I) selon l'une des revendications 1 ou 2 qui est le 6-méthoxy 8-allyl-2-styryl-4H-1 benzopyrane 4-one, ses isomères.

**11.** Procédé de préparation de composé de formule (I) selon l'une des revendications 1 ou 2 qui est la 4-méthoxy-7-(3,4-diméthoxystyryl)-5H-furo(3,2-g)benzopyran-5-one et ses isomères.

Office européen des brevets

# RAPPORT DE RECHERCHE EUROPEENNE

Numero de la demande

EP 91 40 1111

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| Y | EP-A-283761 (TAKEDA CHEMICAL INDUSTRIES)<br>* page 22; revendications 1, 5 *<br>--- | 1, 13 | C07D311/22<br>A61K31/35<br>C07D493/04<br>//(C07D493/04, 311:00,307:00) |
| Y | EP-A-237986 (SHUDO,KOICHI,PROF.DR.)<br>* page 3, alinéa 3 - page 4 *<br>--- | 1, 13 | |
| Y,D | EP-A-237166 (THE STATE OF OREGON ACTING BY AND THROUGH THE OREGON STATE BOARD OF)HIGHER EDUCATION ON BEHALF OF OREGON STATE UNIVERSITY)<br>* page 9; revendications 1, 4 *<br>--- | 1, 13 | |
| Y | CHEMICAL ABSTRACTS, vol. 111, no. 3, 17 juillet 1989<br>Columbus, Ohio, USA<br>Gohar,Abdel-Kerim M.N. et al.: "Reactions with khellin and visnagin.II.Synthesis of several new styrylfurochromones and their fused benz-substituted derivatives of expected biolo"-gical activities.& Egypt.J.Pharm.Sci.1988,vol.29 no.1-4,pages 145-150.<br>page 592; colonne 2; ref. no. 23280R<br>* abrégé *<br>----- | 1, 13 | **DOMAINES TECHNIQUES RECHERCHES (Int. Cl.5 )**<br>C07D311/00<br>C07D493/00 |

*Le présent rapport a été établi pour toutes les revendications*

| Lieu de la recherche | Date d'achèvement de la recherche | Examinateur |
|---|---|---|
| BERLIN | 12 JUILLET 1991 | KYRIAKAKOU, G. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)